# EUROPEAN PATENT APPLICATION

(11) **EP 1 852 419 A1**
(43) Date of publication of application: **07.11.2007**
(21) Application number: 06712415.6
(22) Date of filing: 26.01.2006
(51) Int. Cl.: C07D 207/16

(54) **METHOD FOR PRODUCING FLUORINATED PROLINE DERIVATIVE**

(30) Priority: 27.01.2005 JP 2005020224
(71) Applicant: Mitsui Chemicals, Inc., Tokyo 105-7117 (JP)
(72) Inventor: TSUNODA, Hidetoshi, aura-shi, Chiba, 2990265 (JP); SUZUKI, Tsuneji, aura-shi, Chiba, 2990265 (JP)
(74) Representative: Stuart, Ian Alexander
(86) International application number: PCT/JP2006/301233
(87) International publication number: WO 2006/080401

(57) **Abstract**

An object of the present invention is to provide a method for efficiently producing a high-purity fluorinated proline derivative with high yield. A method tor producing a compound represented by the general formula (2), 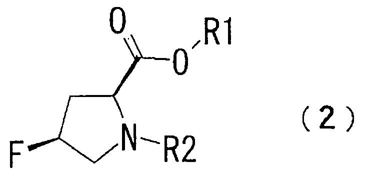 (In the formula, R1 represents an unsubstituted or substituted alkyl group having 1 to 6 carbon atoms or the like, and R2 represents an unsubstituted or substituted alkyl group having 1 to 6 carbon atoms or the like, and R3 represents a hydroxyl group or the like) wherein a compound represented by the general formula (1) 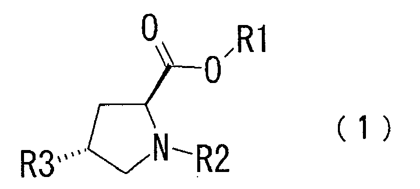 (In the formula, R1 and R2 are defined as in the general formula (1)) is reacted with a fluorination agent to obtain a reaction product containing the compound represented by the general formula (2) and a by-products; and bringing chlorine, bromine, iodine, hypochlorous acid, hypochlorite, hypobromous acid, hypobromite or N-halogenosuccinimide into action on the reaction product to chemically change the by-product.

## Description

### [TECHNICAL FIELD]

The present invention relates to a method for producing a fluorinated proline derivative useful in a wide field including materials for pharmaceuticals, agrochemicals, cosmetics and the like.

### [BACKGROUND ART]

In recent years, a fluorinated proline derivative has drawn attention as a useful compound having bioactivity. For example, in the field of pharmaceuticals, the use of the fluorinated proline derivative has been studied as an important key intermediate of a novel diabetic medicine having a dipeptidyl peptidase IV inhibiting property (see Patent Document 1) or a novel antithrombotic agent having antithrombin activity (see Patent Document 2).

As a conventional method for producing a fluorinated proline derivative, a method for producing from the corresponding hydroxyproline derivative is known. For example, there is known a method for producing fluorinated proline by converting hydroxyproline to a sulfonate ester of hydroxyproline, and then reacting with potassium fluoride to produce fluorinated proline (see Non-patent Document 1). However, fluorinated proline may not be produced stereoselectively by those methods.

In addition, there is known a method for producing a fluorinated proline derivative by reacting a hydroxyproline derivative with (diethylamino)sulfur trifluoride (hereinafter abbreviated as "DAST") (see Non-patent Document 2). This method has a higher reaction yield compared to the above-mentioned method and is useful for stereoselectively producing a fluorinated proline derivative, however, the production method is not described in detail in the documents describing this method.
Patent Document 1: WO2004020407
Patent Document 2: WO2004032834
Non-Patent Document 1: Biochemistry 1965, 4 (11), 2507-2513
Non-Patent Document 2: Tetrahedron Letter 1998, 39, 1169-1172

### [DISCLOSURE OF THE INVENTION]

The present inventors have studied a reaction using the above-mentioned DAST in the process of investigating various methods for producing a fluorinated proline derivative using various fluorination agents. During the investigation, the present inventors have tried to obtain a high-purity fluorinated proline derivative by directly crystallizing the fluorinated proline derivative from a reaction mixture followed by recovering the fluorinated proline derivative. However, it was revealed that it was difficult to obtain a fluorinated proline derivative in high purity by a recrystallization method which is a simple purification method.

The present invention has been made in light of the above situations, and an objective of the present invention is to provide a method for efficiently producing a high-purity fluorinated proline derivative in which the fluorinated proline derivative may be obtained with high yield.

As a result of pursuing the cause of the difficulty to improve the purity of the fluorinated proline derivative, the present inventors found that the cause results from a by-product generated in the reaction of the hydroxyproline derivative with DAST and the by-product was difficult to be separated by the recrystallization from the fluorinated proline derivative which is the target product.

The present inventors have further studied and found out that the above-mentioned by-product is almost not mixed in the final product (a fluorinated proline derivative) by reacting a hydroxyproline derivative with a fluorination agent followed by treating the resulting reaction product with bromine, hypochlorite, N-halogenosuccinimide and the like, and thus completed the present invention.

In other words, the present invention relates to a method for producing a fluorinated proline derivative, a method for purifying a fluorinated proline derivative, and a fluorinated proline derivative, as described in the following.
[1] A method for producing a compound represented by the general formula (2) wherein a compound represented by the formula (1) is reacted with a fluorination agent to obtain a reaction product containing the compound represented by the general formula (2), the compound represented by the general formula (3) and the compound represented by the general formula (4), and then chlorine, bromine, iodine, hypochlorous acid, hypochlorite, hypobromous acid, hypobromite or N-halogenosuccinimide is brought into action on the reaction product to chemically change the compounds represented by the general formula (3) and the general formula (4), wherein the general formulas (1), (2), (3) and (4) represent as the following, respectively: 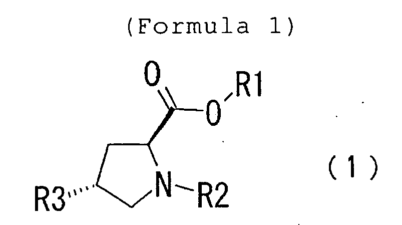 (In the formula, R1 represents an unsubstituted or substituted alkyl group having 1 to 6 carbon atoms, an unsubstituted or substituted benzyl group or an unsubstituted or substituted aryl group. R2 represents an unsubstituted or substituted alkyl group having 1 to 6 carbon atoms, an unsubstituted or substituted benzyl group, an unsubstituted or substituted aryl group or -CO-X, and X represents an unsubstituted or substituted alkyl group having 1 to 6 carbon atoms, a trifluoromethyl group, an unsubstituted or substituted aryl group, an unsubstituted or substituted alkoxy group having 1 to 6 carbon atoms, an unsubstituted or substituted benzyloxy group, or an unsubstituted or substituted fluorenylmethoxy group. R3 represents a hydroxyl group, an unsubstituted or substituted alkylsulfonyloxy group having 1 to 7 carbon atoms, an unsubstituted or substituted arylsulfonyloxy group or a trifluoromethanesulfonyloxy group), 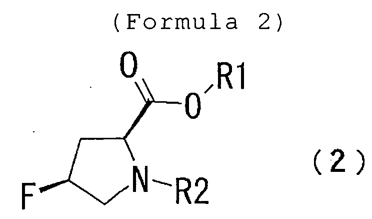 (In the formula, R1 and R2 are defined as in the general formula (1)), 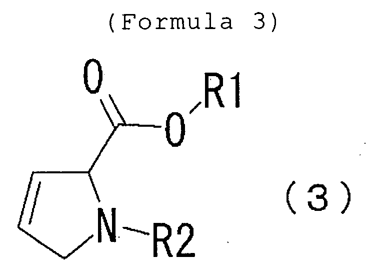 (In the formula, R1 and R2 are defined as in the general formula (1)), 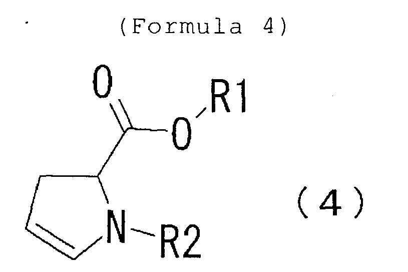 (In the formula, R1 and R2 are defined as in the general formula (1)).
[2] A method for producing a compound represented by the general formula (5) wherein a compound represented by the formula (1) is reacted with a fluorination agent to obtain a reaction product containing the compound represented by the general formula (2), the compound represented by the general formula (3) and the compound represented by the general formula (4), and then the following treatments (A) and (B) are performed on the reaction product obtained by the preceding reaction (the order of performing (A) and (B) may be reversed), followed by recovering the compound represented by the general formula (5) :
   (A) a treatment of converting the carboxylic acid ester portion containing R1 in the general formula (2), general formula (3) and general formula (4) to a carboxylic acid or a salt thereof,
   (B) a treatment of bringing chlorine, bromine, iodine, hypochlorous acid, hypochlorite, hypobromous acid, hypobromite or N-halogenosuccinimide into action on the reaction product to chemically change the compounds represented by the general formula (3) and the general formula (4), wherein the general formulas (1), (2), (3), (4) and (5) represent as the following, respectively: 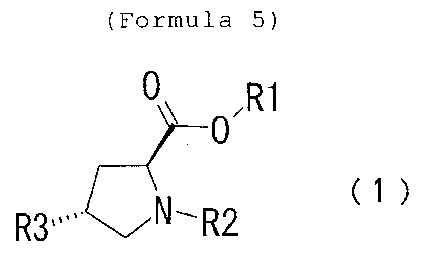 (In the formula, R1 represents an unsubstituted or substituted alkyl group having 1 to 6 carbon atoms, an unsubstituted or substituted benzyl group or an unsubstituted or substituted aryl group. R2 represents an unsubstituted or substituted alkyl group having 1 to 6 carbon atoms, an unsubstituted or substituted benzyl group, an unsubstituted or substituted aryl group or -CO-X, and X represents an unsubstituted or substituted alkyl group having 1 to 6 carbon atoms, a trifluoromethyl group, an unsubstituted or substituted aryl group, an unsubstituted or substituted alkoxy group having 1 to 6 carbon atoms, an unsubstituted or substituted benzyloxy group, or an unsubstituted or substituted fluorenylmethoxy group. R3 represents a hydroxyl group, an unsubstituted or substituted alkylsulfonyloxy group having 1 to 7 carbon atoms, an unsubstituted or substituted arylsulfonyloxy group or a trifluoromethanesulfonyloxy group), 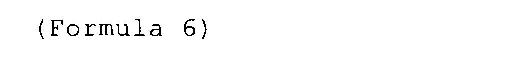 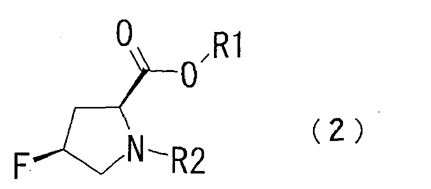 (In the formula, R1 and R2 are defined as in the general formula (1)), 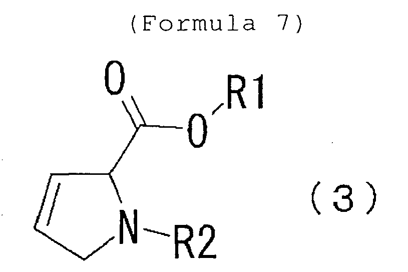 (In the formula, R1 and R2 are defined as in the general formula (1)), 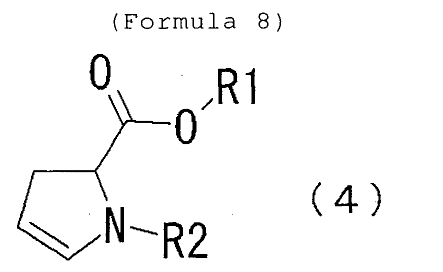 (In the formula, R1 and R2 are defined as in the general formula (1)), 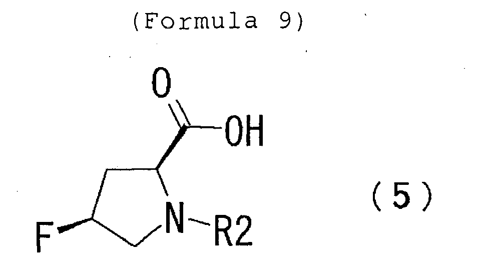 (In the formula, R2 is defined as in the general formula (1)).
[3] A method for producing a compound represented by the general formula (10) wherein a compound represented by the formula (9) is reacted with a fluorination agent to obtain a reaction product containing the compound represented by the general formula (10), the compound represented by the general formula (3) and the compound represented by the general formula (4), and then chlorine, bromine, iodine, hypochlorous acid, hypochlorite, hypobromous acid, hypobromite or N-halogenosuccinimide is brought into action on the reaction product to chemically change the compounds represented by the general formula (3) and the general formula (4), wherein the general formulas (9), (10) (3) and (4) represent as the following, respectively: 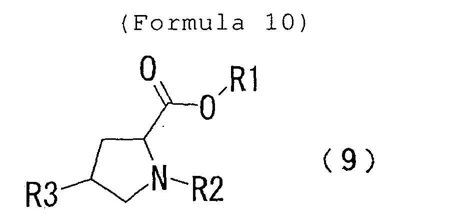 (In the formula, R1 represents an unsubstituted or substituted alkyl group having 1 to 6 carbon atoms, an unsubstituted or substituted benzyl group or an unsubstituted or substituted aryl group. R2 represents an unsubstituted or substituted alkyl group having 1 to 6 carbon atoms, an unsubstituted or substituted benzyl group, an unsubstituted or substituted aryl group or -CO-X, and X represents an unsubstituted or substituted alkyl group having 1 to 6 carbon atoms, a trifluoromethyl group, an unsubstituted or substituted aryl group, an unsubstituted or substituted alkoxy group having 1 to 6 carbon atoms, an unsubstituted or substituted benzyloxy group, or an unsubstituted or substituted fluorenylmethoxy group. R3 represents a hydroxyl group, an unsubstituted or substituted alkylsulfonyloxy group having 1 to 7 carbon atoms, an unsubstituted or substituted arylsulfonyloxy group or a trifluoromethanesulfonyloxy group), 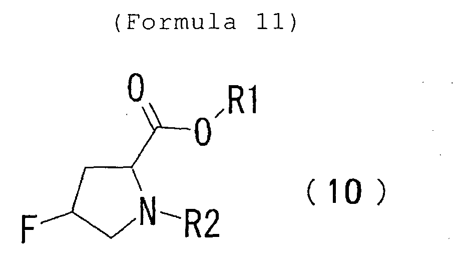 (In the formula, R1 and R2 ar defined as in the general formula (9)), 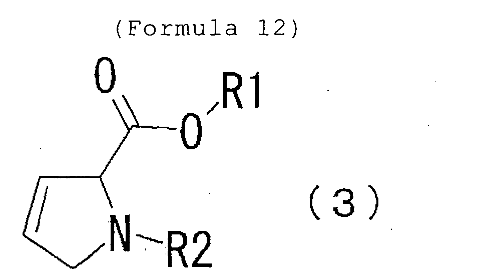 (In the formula, R1 and R2 are defined as in the general formula (9)), 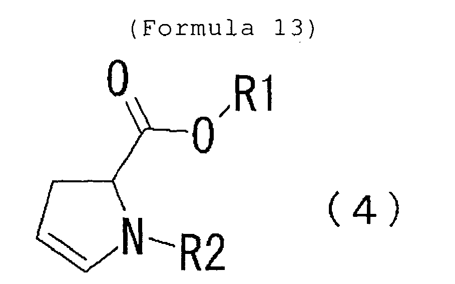 (In the formula, R1 and R2 are defined as in the general formula (9)).
[4] A method for producing a compound represented by the general formula (12) wherein a compound represented by the formula (9) is reacted with a fluorination agent to obtain a reaction product containing the compound represented by the general formula (10), the compound represented by the general formula (3) and the compound represented by the general formula (4), and then the following treatments (A) and (B) are performed on the reaction product to recover the compound represented by the general formula (12) (the order of performing (A) and (B) may be reversed):
   (A) a treatment of converting the carboxylic acid ester portion containing R1 in the general formula (10), the general formula (3) and the general formula (4) to a carboxylic acid or a salt thereof,
   (B) a treatment of bringing chlorine, bromine, iodine, hypochlorous acid, hypochlorite, hypobromous acid, hypobromite or N-halogenosuccinimide into action on the reaction product to chemically change the compounds represented by the general formula (3) and the general formula (4), wherein the general formulas (9), (10), (3), (4) and (12) represent as the following, respectively: 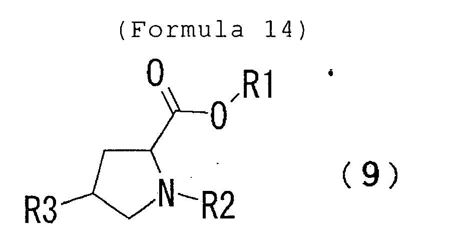 (In the formula, R1 represents an unsubstituted or substituted alkyl group having 1 to 6 carbon atoms, an unsubstituted or substituted benzyl group or an unsubstituted or substituted aryl group. R2 represents an unsubstituted or substituted alkyl group having 1 to 6 carbon atoms, an unsubstituted or substituted benzyl group, an unsubstituted or substituted aryl group or -CO-X, and X represents an unsubstituted or substituted alkyl group having 1 to 6 carbon atoms, a trifluoromethyl group, an unsubstituted or substituted aryl group, an unsubstituted or substituted alkoxy group having 1 to 6 carbon atoms, an unsubstituted or substituted benzyloxy group, or an unsubstituted or substituted fluorenylmethoxy group. R3 represents a hydroxyl group, an unsubstituted or substituted alkylsulfonyloxy group having 1 to 7 carbon atoms, an unsubstituted or substituted arylsulfonyloxy group or a trifluoromethanesulfonyloxy group), 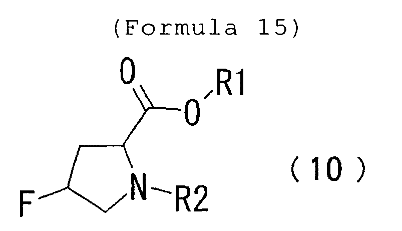 (In the formula, R1 and R2 are defined as in the general formula (9)), 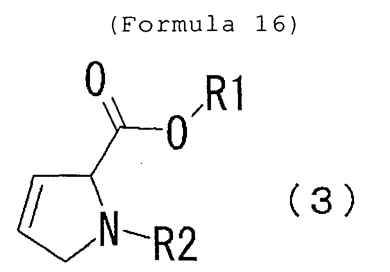 (In the formula, R1 and R2 are defined as in the general formula (9)), 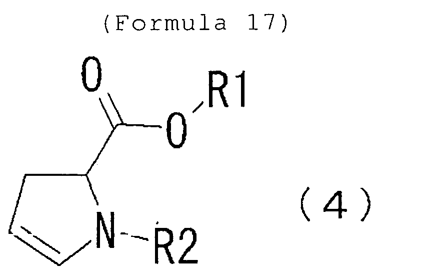 (In the formula, R1 and R2 are defined as in the general formula (9)), 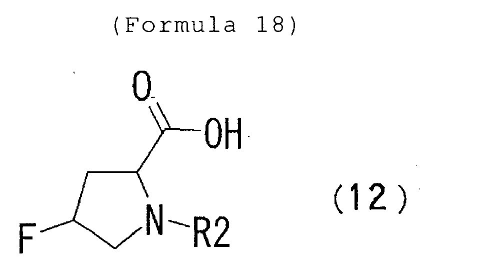 (In the formula, R2 is defined as in the general formula (9)).
[5] A method for purifying a compound represented by the general formula (13) wherein the following treatments (A) and (B) are performed on a mixture containing compounds represented by the general formula (13), the general formula (14) and the general formula (15) (with the proviso that the order of performing (A) and (B) may be reversed, (A) is not required where R10 is a hydrogen atom and (A) is performed as needed where R10 is other than a hydrogen atom), followed by recovering the compound represented by the general formula (13):
   (A) a treatment of converting the carboxylic acid ester portion containing R10 in the general formula (13), the general formula (14) and the general formula (15) to a carboxylic acid or a salt thereof,
   (B) a treatment of bringing chlorine, bromine, iodine, hypochlorous acid, hypochlorite, hypobromous acid, hypobromite or N-halogenosuccinimide into action on the mixture to chemically change the compounds represented by the general formula (14) and the general formula (15), wherein the general formulas (13), (14) and (15) represent as the following, respectively: 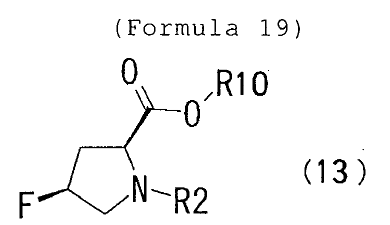 (In the formula, R10 represents a hydrogen atom, an unsubstituted or substituted alkyl group having 1 to 6 carbon atoms, an unsubstituted or substituted benzyl group or an unsubstituted or substituted aryl group. R2 represents an unsubstituted or substituted alkyl group having 1 to 6 carbon atoms, an unsubstituted or substituted benzyl group, an unsubstituted or substituted aryl group or -CO-X, and X represents an unsubstituted or substituted alkyl group having 1 to 6 carbon atoms, a trifluoromethyl group, an unsubstituted or substituted aryl group, an unsubstituted or substituted alkoxy group having 1 to 6 carbon atoms, an unsubstituted or substituted benzyloxy group, or an unsubstituted or substituted fluorenylmethoxy group), 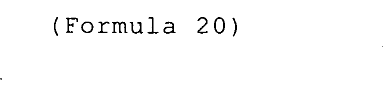 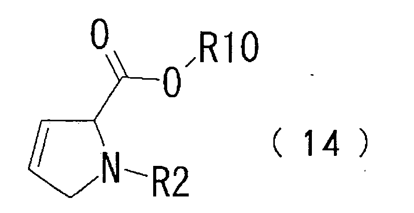 (In the formula, R2 and R10 are defined as in the general formula (13)), 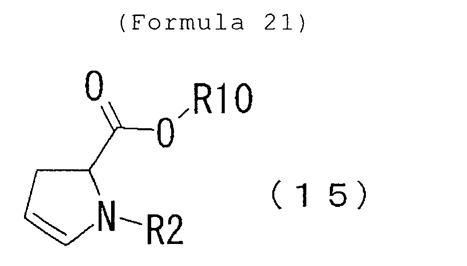 (In the formula, R2 and R10 are defined as in the general formula (13)).
[6] A method for purifying a compound represented by the general formula (17) wherein the following treatments (A) and (B) are performed on a mixture containing the compound represented by the general formula (17), the compound represented by the general formula (14) and the compound represented by the general formula (15) (with the proviso that the order of performing (A) and (B) may be reversed, (A) is not required where R10 is a hydrogen atom and (A) is performed as needed where R10 is other than a hydrogen atom), followed by recovering the compound represented by the general formula (17):
   (A) a treatment of converting the carboxylic acid ester portion containing R10 in the general formula (17), the general formula (14) and the general formula (15) to a carboxylic acid or a salt thereof,
   (B) a treatment of bringing chlorine, bromine, iodine, hypochlorous acid, hypochlorite, hypobromous acid, hypobromite or N-halogenosuccinimide into action on the mixture to chemically change the compounds represented by the general formula (14) and the general formula (15), wherein the general formulas (17), (14) and (15) represent as the following, respectively: 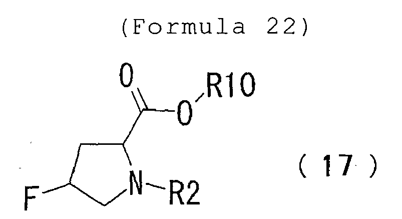 (In the formula, R10 represents a hydrogen atom, an unsubstituted or substituted alkyl group having 1 to 6 carbon atoms, an unsubstituted or substituted benzyl group or an unsubstituted or substituted aryl group. R2 represents an unsubstituted or substituted alkyl group having 1 to 6 carbon atoms, an unsubstituted or substituted benzyl group, an unsubstituted or substituted aryl group or -CO-X, and X represents an unsubstituted or substituted alkyl group having 1 to 6 carbon atoms, a trifluoromethyl group, an unsubstituted or substituted aryl group, an unsubstituted or substituted alkoxy group having 1 to 6 carbon atoms, an unsubstituted or substituted benzyloxy group, or an unsubstituted or substituted fluorenylmethoxy group), 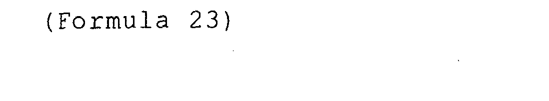 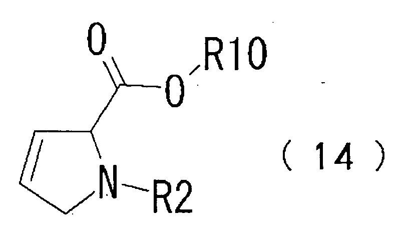 (In the formula, R2 and R10 are defined as in the general formula (17)), 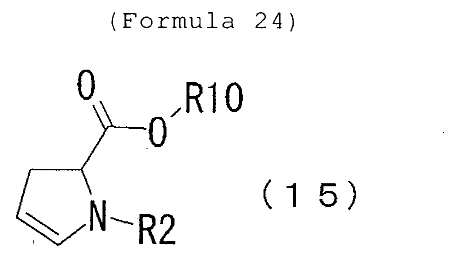 (In the formula, R2 and R10 are defined as in the general formula (17)).
[7] A compound represented by the general formula (21) in which the content of a compound represented by the general formula (19) and a compound represented by the general formula (20) is less than 0.1 % by weight, respectively, with respect to the compound represented by the general formula (21), wherein the general formulas (19), (20) and (21) represent as the following, respectively: 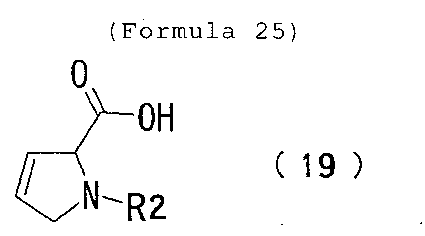 (In the formula, R2 represents an unsubstituted or substituted alkyl group having 1 to 6 carbon atoms, an unsubstituted or substituted benzyl group, an unsubstituted or substituted aryl group or -CO-X, and X represents an unsubstituted or substituted alkyl group having 1 to 6 carbon atoms, a trifluoromethyl group, an unsubstituted or substituted aryl group, an unsubstituted or substituted alkoxy group having 1 to 6 carbon atoms, an unsubstituted or substituted benzyloxy group or an unsubstituted or substituted fluorenylmethoxy group), 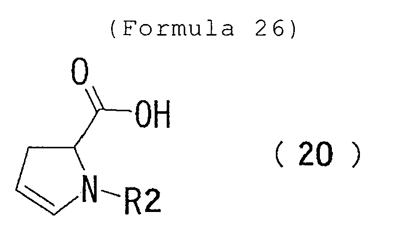 (In the formula, R2 represents an unsubstituted or substituted alkyl group having 1 to 6 carbon atoms, an unsubstituted or substituted benzyl group, an unsubstituted or substituted aryl group or -CO-X, and X represents an unsubstituted or substituted alkyl group having 1 to 6 carbon atoms, a trifluoromethyl group, an unsubstituted or substituted aryl group, an unsubstituted or substituted alkoxy group having 1 to 6 carbon atoms, an unsubstituted or substituted benzyloxy group or an unsubstituted or substituted fluorenylmethoxy group), 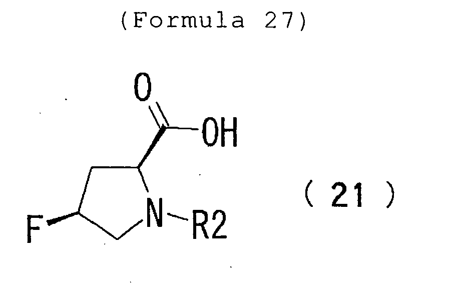 (In the formula, R2 represents an unsubstituted or substituted alkyl group having 1 to 6 carbon atoms, an unsubstituted or substituted benzyl group, an unsubstituted or substituted aryl group or -CO-X, and X represents an unsubstituted or substituted alkyl group having 1 to 6 carbon atoms, a trifluoromethyl group, an unsubstituted or substituted aryl group, an unsubstituted or substituted alkoxy group having 1 to 6 carbon atoms, an unsubstituted or substituted benzyloxy group or an unsubstituted or substituted fluorenylmethoxy group).
[8] A compound represented by the general formula (22) in which the content of a compound represented by the general formula (19) and a compound represented by the general formula (20) is less than 0.1 % by weight, respectively, with respect to the compound represented by the general formula (22), wherein the general formulas (19), (20) and (22) represent as the following, respectively: 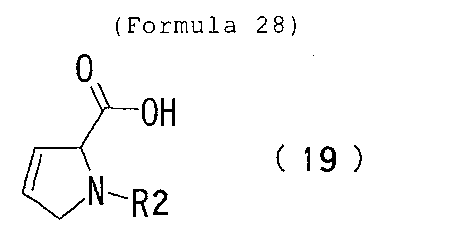 (In the formula, R2 represents an unsubstituted or substituted alkyl group having 1 to 6 carbon atoms, an unsubstituted or substituted benzyl group, an unsubstituted or substituted aryl group or -CO-X, and X represents an unsubstituted or substituted alkyl group having 1 to 6 carbon atoms, a trifluoromethyl group, an unsubstituted or substituted aryl group, an unsubstituted or substituted alkoxy group having 1 to 6 carbon atoms, an unsubstituted or substituted benzyloxy group or an unsubstituted or substituted fluorenylmethoxy group), 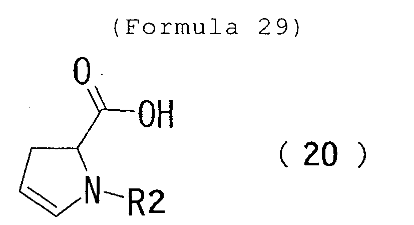 (In the formula, R2 represents an unsubstituted or substituted alkyl group having 1 to 6 carbon atoms, an unsubstituted or substituted benzyl group, an unsubstituted or substituted aryl group or -CO-X, and X represents an unsubstituted or substituted alkyl group having 1 to 6 carbon atoms, a trifluoromethyl group, an unsubstituted or substituted aryl group, an unsubstituted or substituted alkoxy group having 1 to 6 carbon atoms, an unsubstituted or substituted benzyloxy group or an unsubstituted or substituted fluorenylmethoxy group), 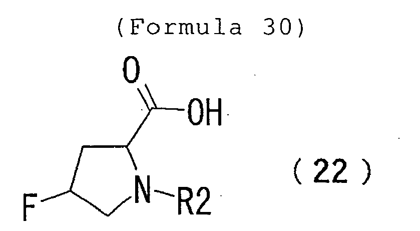 (In the formula, R2 represents an unsubstituted or substituted alkyl group having 1 to 6 carbon atoms, an unsubstituted or substituted benzyl group, an unsubstituted or substituted aryl group or -CO-X, and X represents an unsubstituted or substituted alkyl group having 1 to 6 carbon atoms, a trifluoromethyl group, an unsubstituted or substituted aryl group, an unsubstituted or substituted alkoxy group having 1 to 6 carbon atoms, an unsubstituted or substituted benzyloxy group or an unsubstituted or substituted fluorenylmethoxy group).

According to the present invention, a high-purity fluorinated proline derivative may be obtained with high efficiency and high yield.

The present invention is a novel method for efficiently producing a fluorinated proline derivative that is expected to be used in the applications as a material for pharmaceuticals, agrochemicals, cosmetics and the like, from an industrial viewpoint, and is extremely useful.

Firstly, the compounds represented by the above-mentioned general formula (1) and the general formula (9) will be explained in detail.
R1 represents an unsubstituted or substituted alkyl group having 1 to 6 carbon atoms, an unsubstituted or substituted benzyl group or an unsubstituted or substituted aryl group.
The "unsubstituted or substituted alkyl group having 1 to 6 carbon atoms" means an unsubstituted alkyl group having 1 to 6 carbon atoms or an alkyl group having 1 to 6 carbon atoms in which an arbitrary hydrogen atom is substituted with a substituent.
The alkyl group having 1 to 6 carbon atoms includes methyl group, ethyl group, isopropyl group, n-butyl group, tert-butyl group, n-pentyl group, n-hexyl group, cyclohexyl group, allyl group and the like.
In addition, the substituent of the alkyl group having 1 to 6 carbon atoms includes phenyl group, hydroxyl group, alkoxy group such as methoxy group, benzyloxy group, methoxyethoxy group and the like, phenoxy group, nitro group, amino group, amido group, carboxyl group, alkoxycarbonyl group, phenoxycarbonyl group, halogen atom such as fluorine atom, chlorine atom, bromine atom or iodine atom, and the like.

The "unsubstituted or substituted benzyl group" in R1 means an unsubstituted benzyl group or a benzyl group in which an arbitrary hydrogen atom is substituted with a substituent.
The substituent of the benzyl group includes alkyl group, phenyl group, hydroxyl group, alkoxy group such as methoxy group, benzyloxy group, methoxyethoxy group and the like, phenoxy group, nitro group, amino group, amido group, carboxyl group, alkoxycarbonyl group, phenoxycarbonyl group, halogen atom such as fluorine atom, chlorine atom, bromine atom or iodine atom, and the like. For example, a benzhydryl group, trityl group and dimethoxytrityl group also belong to the category of a "substituted benzyl group".

The "unsubstituted or substituted aryl group" in R1 means an unsubstituted aryl group or an aryl group in which an arbitrary hydrogen atom is substituted with a substituent.
The aryl group includes phenyl group, naphthyl group, biphenyl group, anthracenyl group, pyridyl group, quinolyl group, furyl group, thienyl group and the like.
In addition, the substituent of the aryl group includes alkyl group, hydroxyl group, alkoxy group such as methoxy group, benzyloxy group, methoxyethoxy group and the like, phenoxy group, nitro group, amino group, amido group, carboxyl group, alkoxycarbonyl group, phenoxycarbonyl group, halogen atom such as fluorine atom, chlorine atom, bromine atom or iodine atom, and the like.
The above-mentioned groups represented by R1 may be linear or branched.

R2 represents an unsubstituted or substituted alkyl group having 1 to 6 carbon atoms, an unsubstituted or substituted benzyl group, an unsubstituted or substituted aryl group or -CO-X, and X represents an unsubstituted or substituted alkyl group having 1 to 6 carbon atoms, a trifluoromethyl group, an unsubstituted or substituted aryl group, an unsubstituted or substituted alkoxy group having 1 to 6 carbon atoms, an unsubstituted or substituted benzyloxy group or an unsubstituted or substituted fluorenylmethoxy group.
The above-mentioned "unsubstituted or substituted alkyl group having 1 to 6 carbon atoms", "unsubstituted or substituted benzyl group" and "unsubstituted or substituted aryl group" have the same meaning as the "unsubstituted or substituted alkyl group having 1 to 6 carbon atoms", "unsubstituted or substituted benzyl group" and "unsubstituted or substituted aryl group" in R1 of the general formula (9).

The "unsubstituted or substituted alkoxy group having 1 to 6 carbon atoms" in R2 means an unsubstituted alkoxy group having 1 to 6 carbon atoms or an alkoxy group having 1 to 6 carbon atoms in which an arbitrary hydrogen atom is substituted by a substituent. The alkoxy group having 1 to 6 carbon atoms includes methoxy group, ethoxy group, isopropoxy group, n-butoxy group, tert-butoxy group, n-pentyloxy group, n-hexyloxy group, cyclohexyloxy group, allyloxy group and the like.
In addition, the substituent of the alkoxy group having 1 to 6 carbon atoms includes phenyl group, hydroxyl group, alkoxy group such as methoxy group, ethoxy group, benzyloxy group, and the like, phenoxy group, nitro group, amino group, amido group, carboxyl group, alkoxycarbonyl group, phenoxycarbonyl group, halogen atom such as fluorine atom, chlorine atom, bromine atom or iodine atom, and the like.

The "unsubstituted or substituted benzyloxy group" means an unsubstituted benzyloxy group or a benzyloxy group in which an arbitrary hydrogen atom is substituted by a substituent.
The substituent of the benzyloxy group includes alkyl group, phenyl group, hydroxyl group, alkoxy group such as methoxy group, benzyloxy group, methoxyethoxy group and the like, phenoxy group, nitro group, amino group, amido group, carboxyl group, alkoxycarbonyl group, phenoxy carbonyl group, halogen atom such as fluorine atom, chlorine atom, bromine atom or iodine atom, and the like. For example, a benzhydryloxy group, trityloxy group, dimethoxytrityloxy group and the like also belong to the category of a "substituted benzyloxy group".

The "unsubstituted or substituted fluorenylmethoxy group" means an unsubstituted fluorenylmethoxy group or a fluorenylmethoxy group in which an arbitrary hydrogen atom is substituted by a substituent.
The substituent of the fluorenylmethoxy group includes alkyl group, phenyl group, hydroxyl group, alkoxy group such as methoxy group, benzyloxy group, methoxyethoxy group and the like, phenoxy group, nitro group, amino group, amido group, carboxyl group, alkoxycarbonyl group, phenoxycarbonyl group, halogen atom such as fluorine atom, chlorine atom, bromine atom and iodine atom, and the like.
The above-mentioned groups represented by R2 may be linear or branched.

R3 represents hydroxyl group, an unsubstituted or substituted alkylsulfonyloxy group having 1 to 7 carbon atoms, an unsubstituted or substituted arylsulfonyloxy group or a trifluoromethanesulfonyloxy group.
The above-mentioned "unsubstituted or substituted alkylsulfonyloxy group having 1 to 6 carbon atoms" in R3 means an unsubstituted alkylsulfonyloxy group having 1 to 6 carbon atoms or an alkylsulfonyloxy group having 1 to 6 carbon atoms in which an arbitrary hydrogen atom is substituted by a substituent.
The alkylsulfonyloxy group having 1 to 6 carbon atoms includes methanesulfonyloxy group, ethanesulfonyloxy group, n-propanesulfonyloxy group, isopropanesulfonyloxy group, n-butanesulfonyloxy group, tert-butanesulfonyloxy group, n-pentanesulfonyloxy group, n-hexanesulfonyloxy group, cyclohexanesulfonyloxy group, allylsulfonyloxy group and the like.
In addition, the substituent of the alkylsulfonyloxy group having 1 to 6 carbon atoms includes phenyl group, hydroxyl group, alkoxy group such as methoxy group, benzyloxy group, methoxyethoxy group and the like, phenoxy group, nitro group, amino group, amido group, carboxyl group, alkoxycarbonyl group, phenoxycarbonyl group, halogen atom such as fluorine atom, chlorine atom, bromine atom and iodine atom, and the like.

The "unsubstituted or substituted arylsulfonyloxy group" in R3 means an unsubstituted arylsulfonyloxy group or an arylsulfonyloxy group in which an arbitrary hydrogen atom is substituted by a substituent.
The arylsulfonyloxy group includes benzenesulfonyloxy group, naphthalenesulfonyloxy group, biphenylsulfonyloxy group, anthracenesulfonyloxy group and the like. In addition, the substituent of the aryl group includes alkyl group, hydroxyl group, alkoxy group such as methoxy group, benzyloxy group, methoxyethoxy group or the like, phenoxy group, nitro group, amino group, amido group, carboxyl group, alkoxycarbonyl group, phenoxycarbonyl group, halogen atom such as fluorine atom, chlorine atom, bromine atom and iodine atom, and the like.
The above-mentioned groups represented by R3 may be linear or branched.
In the present invention, as a compound represented by the general formula (9), a compound represented by the general formula (11) in which R3 is hydroxyl group, may be used. 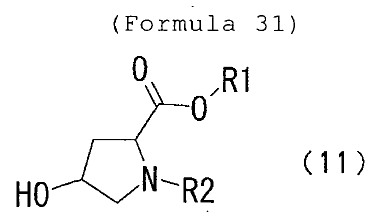 (In the formula, R1 and R2 are defined as in the general formula (9))
The fluorination at the bonding site of hydroxyl group is facilitated by the use of a compound represented by the general formula (11) and a compound represented by the general formula (10) may be obtained with high yield.
In addition, a compound (an optically active substance) represented by the general formula (1) may be used in place of a compound (a racemic modification) represented by the general formula (9),

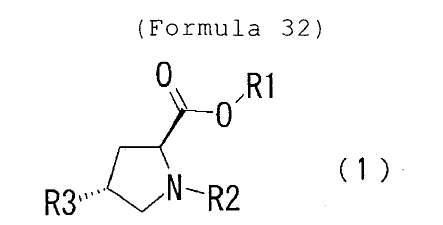

### (In the formula, R1, R2 and R3 are defined as in the above)

In the present invention, as a compound represented by the general formula (1), a compound represented by the general formula (25) in which R3 is hydroxyl group, may be used.

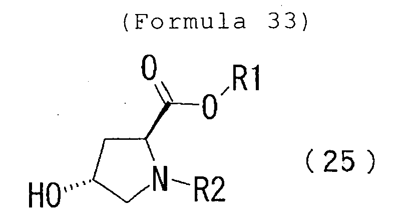

### (In the formula, R1 and R2 are defined as above)

The fluorination at the bonding site of hydroxyl group is facilitated by the use of a compound (R configuration) represented by the general formula (25), and a compound (S configuration) represented by the general formula (2) which is described hereinbelow may be stereoselectively obtained with high yield.

As the compounds represented by the general formulas (9), (11), (1) and (25), commercially available compounds may be used. In addition, the compounds may be readily synthesized from hydroxyproline which is available as a marketed product, using a known method.

In the present invention, as the fluorination agent which is capable of fluorinating a raw material compound represented by the general formula (1) or (9), there may be used, it is not particularly limited, but a compound capable of being reacted with a raw material compound to generate a compound (fluorinated compound) represented by the general formula (2) or (10).

The fluorination agent includes, for example, a compound represented by the general formula (6),

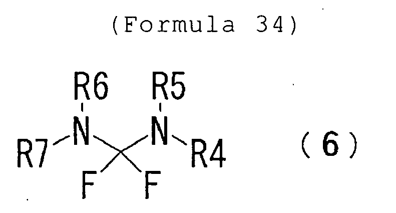

a compound represented by the general formula (7), 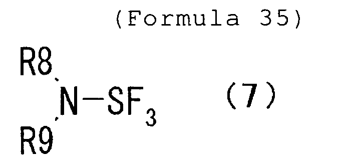 perfluorobutanesulfonyl fluoride (C₄F₉SO₂F), a hydrogen fluoride pyridine complex, 1,1-difluoromethyl-N,N-diisopropylamine, an inorganic salt such as potassium fluoride and cesium fluoride, an ammonium salt such as n-tetrabutylammonium fluoride and the like.

In the general formula (6), R4, R5, R6 and R7 represent an unsubstituted or substituted alkyl group having 1 to 6 carbon atoms, an unsubstituted or substituted benzyl group and an unsubstituted or substituted aryl group, and may be the same or different. In addition, R5 and R6 in the formula may be linked together to form a five-membered ring or a six-membered ring, and R4 and R5, or R6 and R7 may be linked together to form a ring.

In addition, the "unsubstituted or substituted alkyl group having 1 to 6 carbon atoms", "unsubstituted or substituted benzyl group" and "unsubstituted or substituted aryl group" described in R4, R5, R6 and R7 of the general formula (6) have the same meanings as those explained in R1.

In the general formula (7), R8 and R9 represent an unsubstituted or substituted alkyl group having 1 to 6 carbon atoms, an unsubstituted or substituted benzyl group and an unsubstituted or substituted aryl group, and may be the same or different. In addition, R8 and R9 may be linked together to form a ring.

As the compound represented by the general formula (6), there may be mentioned, for example,

2,2-difluoro-1,3-dimethylimidazolidine represented by the following formula (8).

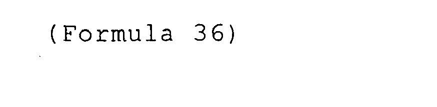

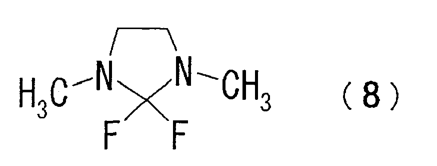

The compound represented by the general formula (7) may be exemplified by (diethylamino)sulfur trifluoride (DAST) and bis(2-methoxyethyl)aminosulfur trifluoride (Deoxo-fluor).

Among the fluorination agents, 2,2-difluoro-1,3-dimethylimidazolidine and DAST are preferable compounds from the viewpoint that a target compound may be stereoselectively produced and the reaction yield is high.

The amount of a fluorination agent used for the reaction with a compound of the general formula (1) or (9) is not particularly limited, but the fluorination agent is typically used in an amount of 1 molar equivalent to 20 molar equivalents (1 molar equivalent or more and 20 molar equivalents or less) with respect to the compound of the general formula (1) or (9).

The reaction of a fluorination agent with a compound of the general formula (1) or (9) may be carried out in a reaction solvent as needed. The reaction solvent used for the reaction is not particularly limited as long as it does not adversely affect the reaction. For example, as a preferred reaction solvent where the fluorination agent used is a compound represented by the general formula (6), 2,2-difluoro-1,3-dimethylimidazolidine, a compound represented by the general formula (7), (diethylamino)sulfur trifluoride (DAST), (2-methoxyethyl)aminosulfur trifluoride (Deoxo-fluor), perfluorobutanesulfonyl fluoride (C₄F₉SO₂F), a hydrogen fluoride pyridine complex and 1, 1-difluoromethyl-N, N-diisopropylamine, there may be mentioned, for example, a hydrocarbon-based solvent such as toluene, xylene, cumene, hexane and the like, a halogen-based solvent such as dichloromethane, chloroform and the like, an ether-based solvent such as diethyl ether, tetrahydrofuran, 1,4-dioxane and the like, and a polar solvent such as 1,3-dimethyl-2-imidazolidinone and the like.
In addition, as a preferred reaction solvent where the fluorination agent used is an inorganic salt such as potassium fluoride and cesium fluoride and an ammonium salt such as n-tetrabutylammonium fluoride, there may be mentioned, for example, water, an alcohol-based solvent such as methanol, ethanol, isopropanol and the like, a hydrocarbon-based solvent such as toluene, xylene, cumene, hexane and the like, a halogen-based solvent such as dichloromethane, chloroform and the like, an ether-based solvent such as diethyl ether, tetrahydrofuran, 1,4-dioxane and the like, an ester-based solvent such as ethyl acetate, butyl acetate and the like, and a nonprotic polar solvent such as N,N-dimethylformamide, dimethylsulfoxide, N-methylpyrolidinone, 1,3-dimethyl-2-imidazolidinone and the like.

The reaction temperature is not particularly limited as long as the reaction proceeds, and the reaction may be carried out typically at a temperature -100°C to the boiling point of the solvent used.

Next, a reaction product obtained by the fluorination reaction is explained.
When a compound represented by the general formula (9) is fluorinated, there may be obtained a reaction product including a compound represented by the general formula (10), a compound represented by the general formula (3) and a compound represented by the general formula (4). Here, the compounds represented by the general formula (3) and the general formula (4) are by-products. 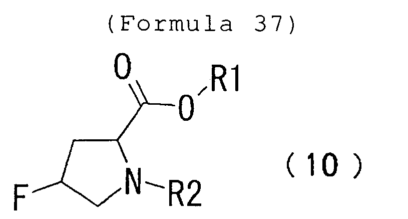 (In the formula, R1 and R2 are defined as in the general formula (9)) 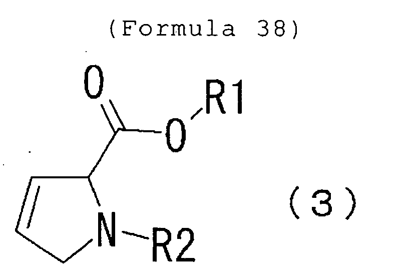 (In the formula, R1 and R2 are defined as in the general formula (9)) 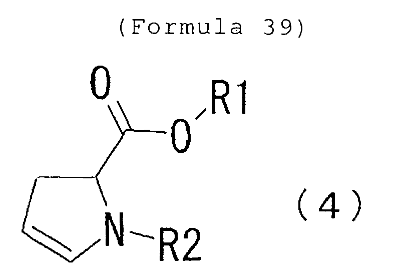 (In the formula, R1 and R2 are defined as in the general formula (9))
Meanwhile, even when a compound represented by the general formula (11) is used as a compound represented by the general formula (9), a similar reaction product may be obtained.
In addition, when a compound (an optically active substance) represented by the general formula (1) is fluorinated, there may be obtained a reaction product including a compound represented by the general formula (2), a compound represented by the general formula (3) and a compound represented by the general formula (4). 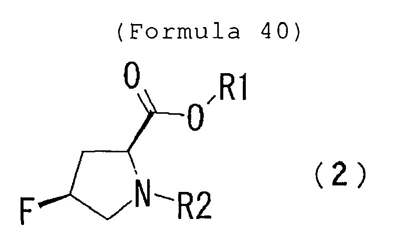 (In the formula, R1 and R2 are defined as in the general formula (1)) 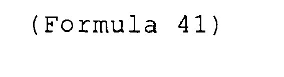 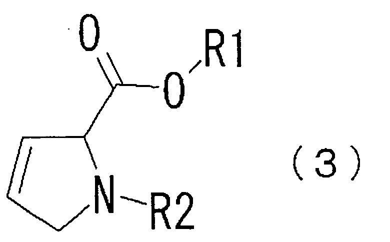 (In the formula, R1 and R2 are defined as in the general formula (1)) 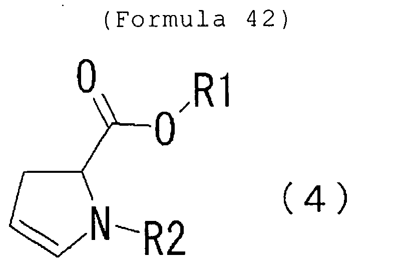 (In the formula, R1 and R2 are defined as in the general formula (1))
In this way, the compounds represented by the general formula (3) and the general formula (4) are generated as by-products in a reaction product obtained by the fluorination reaction.
Such a reaction product may be used for the subsequent reaction after treating with a solution of a base such as potassium hydroxide, for example, by a known method. In addition, a mixture containing a fluorinated compound and by-product (s) may be recovered from a reaction product and used for the subsequent reaction.

A method for recovering a mixture containing a fluorinated compound and by-product(s) is not particularly limited, but a method for distilling the organic solvent out of the organic layer in which these compounds are extracted in a water-immiscible organic solvent including a hydrocarbon-based solvent such as toluene, xylene, cumene, hexane and the like, a halogen-based solvent such as dichloromethane, chloroform and the like, an ether-based solvent such as diethyl ether and the like, and an ester-based solvent such as ethyl acetate, butyl acetate and the like may be exemplified.

The target compound may be produced by first performing the following treatments (A) and (B) on a reaction product including the fluorinated compound and by-product(s), or on a mixture containing the fluorinated compound and by-product(s) recovered from the reaction product, and subsequently by recovering the target compound (a compound represented by the general formula (5) or (12)) from the reaction mixture obtained by performing these treatments. Here, the order of performing (A) and (B) may be reversed. In addition, when the target compound is a compound of the general formula (2) or (10) as needed, it is not required to perform (A) and the target compound may be recovered after performing (B) ;
(A) in each compound represented by the general formula (2) (or the general formula (10)), the general formula (3) and the general formula (4), a treatment of converting the carboxylic acid ester portion containing R1 to carboxylic acid or a salt thereof (hereinafter simply referred to as the "treatment A"), and
(B) a treatment of bringing chlorine, bromine, iodine, hypochlorous acid, hypochlorite, hypobromous acid, hypobromite or N-halogenosuccinimide into action on the reaction product to chemically change the compound represented by the general formula (3) and the general formula (4) (hereinafter simply referred to as the "treatment B").
   Hereinafter, the contents of the treatments (A) and (B) will be explained in more detail.

### [Treatment (A)]

In the treatment (A), the treatment of converting the carboxylic acid ester portion containing R1 of the fluorinated compound and by-product(s) to carboxylic acid or a salt thereof is not particularly limited as long as the carboxylic acid ester portion containing R1 in the fluorinated compound may be changed to carboxylic acid or a salt thereof, but a treatment using a known method, for example, an ester hydrolysis reaction or a hydrogenolysis reaction, are exemplified.

For example, the ester hydrolysis reaction may be carried out under a basic condition or under an acidic condition.

The base usable for enabling a basic condition is not particularly limited, but a hydroxide and the like of an alkali or an alkaline earth metal such as lithium hydroxide, sodium hydroxide, potassium hydroxide, barium hydroxide and the like may be exemplified. The acid usable for enabling an acidic condition is not particularly limited, but an inorganic acid such as hydrochloric acid, hydrobromic acid, sulfuric acid and the like, and an organic acid such as trifluoromethanesulfonic acid, trifluoroacetic acid and the like may be exemplified.

The amount of a base or an acid used for the reaction is not particularly limited, but for example, when a base is used, the base is typically used in an amount of 1 molar equivalent or more with respect to the fluorinated compound, and when an acid is used, the acid is typically used in an amount of 0.001 molar equivalents or more with respect to the fluorinated compound.

The reaction solvent used for the ester hydrolysis reaction is not particularly limited as long as it does not disturb the progress of the reaction. For example, water or an alcohol-based solvent such as methanol, ethanol, isopropanol and the like may be used singly. In addition, a mixed solvent of water and an arbitrary solvent may be used. A reaction solvent which may be used as a mixed solvent with water is not particularly limited. For example, there may be used an alcohol-based solvent such as methanol, ethanol, isopropanol and the like, a hydrocarbon-based solvent such as toluene, xylene, cumene, hexane and the like, a halogen-based solvent such as dichloromethane, chloroform and the like, an ether-based solvent such as diethyl ether, tetrahydrofuran, 1,4-dioxane and the like, and a polar reaction solvent such as N,N-dimethylformamide, dimethylsulfoxide, N-methylpyrolidinone, 1,3-dimethyl-2-imidazolidinone and the like. Further, the reaction solvents mentioned here may be used as a mixed solvent with water at an arbitrary ratio and in an arbitrary combination.

The reaction temperature of the ester hydrolysis reaction is not particularly limited as long as the reaction proceeds, and the reaction may be carried out typically at a temperature from -20°C to the boiling point of the solvent used.

On the other hand, the hydrogenolysis reaction is especially effective where R1 in a compound represented by the general formula (2) (or the general formula (10)) is an "unsubstituted or substituted benzyl group". For example, the hydrogenolysis reaction may be carried out under a hydrogen atmosphere using a carbon-palladium catalyst, a platinum catalyst, Raney nickel and the like as a catalyst.

The hydrogen pressure and the amount of the catalyst used are not particularly limited as long as they do not disturb the progress of the reaction.

The reaction solvent of the hydrogenolysis reaction is not particularly limited as long as it does not disturb the progress of the reaction.
For example, there may be used water, an alcohol-based solvent such as methanol, ethanol, isopropanol and the like, a hydrocarbon-based solvent such as toluene, xylene, cumene, hexane and the like, an ether-based solvent such as diethyl ether, tetrahydrofuran, 1,4-dioxane and the like, an ester-based solvent such as ethyl acetate, butyl acetate and the like, and a nonprotic polar solvent such as N,N-dimethylformamide, dimethylsulfoxide, N-methylpyrolidinone, 1, 3-dimethyl-2-imidazolidinone and the like.

The reaction temperature of the hydrogenolysis reaction is not particularly limited as long as the reaction proceeds, and the reaction may be carried out typically at a temperature from -20°C to the boiling point of the solvent used.

### [Treatment (B)]

The treatment of (B) is a process in which chlorine, bromine, iodine, hypochlorous acid, hypochlorite, hypobromous acid, hypobromite or N-halogenosuccinimide is brought into action on the reaction product including a fluorinated compound and by-product(s) obtained in the above-mentioned treatment (A) to chemically change the by-product(s).
By the treatment (B), the fluorinated compound may be readily recovered and obtained with high yield. In addition, the treatment (B) may be performed in a one-pot step together with a step of converting the carboxylic acid ester portion to carboxylic acid, and is a convenient production process, making it possible to obtain a high-purity fluorinated proline derivative with extremely high efficiency.
The "hypochlorite" used in the treatment (B) includes, for example, lithium hypochlorite, sodium hypochlorite, potassium hypochlorite, calcium hypochlorite and the like. The "hypobromite" includes, for example, sodium hypobromite, potassium hypobromite and the like.

The "N-halogenosuccinimide" includes, for example, N-chlorosuccinimide, N-bromosuccinimide, N-iodosuccinimide and the like.

The amount of chlorine, bromine, iodine, hypochlorous acid, hypochlorite, hypobromous acid, hypobromite or N-halogenosuccinimide used in the reaction is not particularly limited. These compounds may be used in an amount of preferably 0.1 molar equivalents to 10 molar equivalents and more preferably 1 molar equivalent to 3 molar equivalents, based on 1 molar equivalent in total of the compounds represented by the general formula (3) and the general formula (4) which are typical by-products. Where the amount is within the above range, as described hereinbelow, the compounds represented by the general formula (3) and the general formula (4) are chemically changed selectively and thus the fluorinated compound is readily recovered, thereby enabling to prevent the compounds represented by the general formula (3) and the general formula (4) from remaining in the final product. Such effect becomes significant particularly in the above preferred range.

The reaction solvent used in the treatment (B) is not particularly limited as long as it does not disturb the progress of the reaction. For example, there may be used water, an alcohol-based solvent such as methanol, ethanol, isopropanol and the like, a hydrocarbon-based solvent such as toluene, xylene, cumene, hexane and the like, a halogen-based solvent such as dichloromethane, chloroform and the like, an ether-based solvent such as diethyl ether, tetrahydrofuran, 1, 4-dioxane and the like, an ester-based solvent such as ethyl acetate, butyl acetate and the like, and a nonprotic polar solvent such as N,N-dimethylformamide, dimethylsulfoxide, N-methylpyrolidinone, 1, 3-dimethyl-2-imidazolidinone and the like. These solvents may be used singly, or may be used by mixing with an arbitrary mixing ratio.

The pH in performing the treatment (B) is not particularly limited, but is preferably 1 to 14 and more preferably from 4 to 6 from the viewpoint that the fluorinated compound and the target compound (a compound represented by the general formula (5) or (12)) are not decomposed and the progress of the reaction is not disturbed in this range.

The reaction temperature is not particularly limited as long as the reaction proceeds, and is typically -20°C to the boiling point of the solvent used and preferably -20°C to 40°C.
In the treatment (B), the compounds represented by the general formula (3) and the general formula (4) which are by-products are selectively subjected to chemical changes by a combination of the reaction conditions such as the equivalence ratio, pH, reaction temperature and the like appropriately, thus the treatment (B) enables the compounds represented by the general formula (3) and the general formula (4) to be changed to a state in which they may be separated from the compounds represented by the general formula (5) and the general formula (12)'in the recovery treatment described hereinbelow, and as a result brings about a state where the fluorinated compound which is the target product may be recoverable a the reaction product.
" Selectively subjecting the compounds represented by the general formula (3) and the general formula (4) to chemical changes" means that the by-product(s) is(are) preferentially subjected to chemical changes compared to the fluorinated compound and as a result the concentration of the fluorinated compound in a reaction product is increased, not only in the case where the compounds represented by the general formula (3) and the general formula (4) undergo chemical change, but also in the case where even a small amount of the fluorinated compound which is the target compound may be changed.
For this reason, the recovery of the fluorinated compound is facilitated and a high-purity fluorinated proline derivative may be obtained.
By performing the treatment (B), there may be reduced the amount of the compounds represented by the general formula (3) and the general formula (4) which are by-products contained together with the fluorinated compound represented by the general formula (2) (or the general formula (10)) to less than 0.1 % by weight, respectively, based on 100 % by weight of the fluorinated compound. Therefore, the compounds represented by the general formula (3) and the general formula (4) which are by-products hardly remain in the final product (a fluorinated proline derivative).

After the above-mentioned treatment (B) and as needed the treatment (A) are performed, the target compounds (the compounds represented by the general formula (5) or the general formula (12), or the compounds represented by the general formula (2) or the general formula (10)) are recovered from the reaction mixture obtained by performing these treatments.

The method for recovering the above-mentioned target compounds is not particularly limited, and there may be mentioned, for example, a method in which, when the reaction solution is treated with an acid to adjust the pH to the range of 1 to 5 and a compound represented by the target compound is precipitated, the resulting solution is filtered to recover target compound. On the other hand, even when the target compound is not precipitated by adjusting the pH to the range of 1 to 5 by treating the reaction solution with an acid, the target compound may be extracted in a water-immiscible organic solvent, followed by recovering from the reaction solution by a known method such as distilling off the organic solvent.

The acid which may be used in the above-mentioned recovery method is not particularly limited, and includes, for example, an inorganic acid such as hydrochloric acid, sulfuric acid, phosphoric acid and the like, an organic acid such as acetic acid, methanesulfonic acid, trifluoroacetic acid and the like, and others.

The water-immiscible organic solvent which may be used in the above-mentioned recovery method is not particularly limited as long as it is capable of extracting the target compound (a compound represented by the general formula (5) or (12)) from water, and includes, for example, a hydrocarbon-based solvent such as toluene, xylene, cumene, hexane and the like, a halogen-based solvent such as dichloromethane, chloroform and the like, an ether-based solvent such as diethyl ether, tetrahydrofuran, 1,4-dioxane and the like, an ester-based solvent such as ethyl acetate, butyl acetate and the like.
By the above-mentioned method, the target compound containing less amount of the by-products may be obtained.

When the target compound (represented by the general formula (5) or the general formula (12)) is extracted in a water-immiscible organic solvent, the extraction solution is concentrated, and to the resulting concentrated solution is added a solvent which is difficult to dissolve the target compound, for example, toluene, thereby enabling to recover the target compound as powders and crystals. In the above description, the embodiments for recovering a compound represented by the general formula (5) or (12) are explained, however, a compound represented by the general formula (2) or (10) may be recovered also by a known method.

Next, a specific embodiment of a method for producing a fluorinated proline derivative relating to the present invention will be exemplified.

For example, a compound represented by the general formula (11) belonging to a compound represented by the general formula (9)

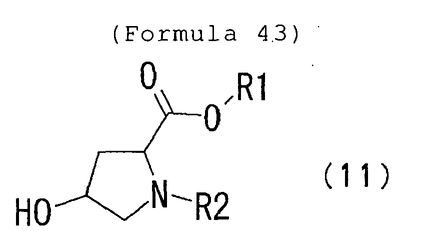

is reacted with the fluorination agent to obtain a reaction product containing the compound represented by the general formula (10), the compound represented by the general formula (3) and the compound represented by the general formula (4), and the above-mentioned treatments (A) and (B) are performed on the reaction product (with the proviso that the order of performing (A) and (B) may be reversed) and subsequently a compound represented by the general formula (12)

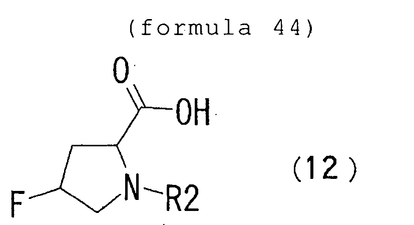

is recovered to produce a compound represented by the general formula (12).

(R1 and R2 in the general formula (11) and the general formula (12) are defined as R1 and R2 in the general formula (9), respectively.)

In addition, a compound represented by the general formula (1)

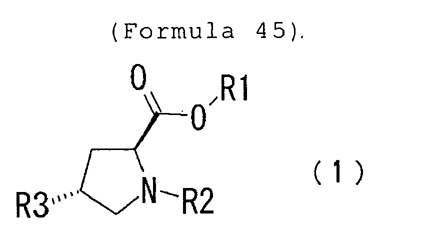

is reacted with the fluorination agent to obtain a reaction product containing the compounds represented by the general formula (2), the compounds represented by the general formula (3) and the compounds represented by the general formula (4), and the above-mentioned treatments (A) and (B) are performed on the reaction product (with the proviso that the order of performing (A) and (B) may be reversed)

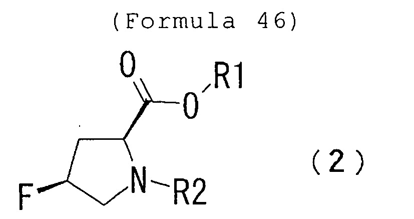

and subsequently a compound represented by the general formula (16) is recovered to produce a compound represented by the general formula (16). In addition, no racemization occurs even when the treatments (A) and (B) are performed on a compound represented by the general formula (2).

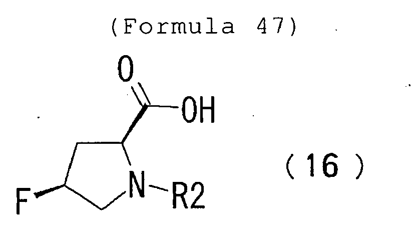

R1 and R2 in the general formula (2) are defined as R1 and R2 in the general formula (1), respectively.

R2 in the general formula (16) is defined as R2 in the general formula (1).

When the treatment (A) is performed on the above-mentioned compound represented by the general formula (3) or the compound represented by the general formula (4),

a compound represented by the general formula (23) or

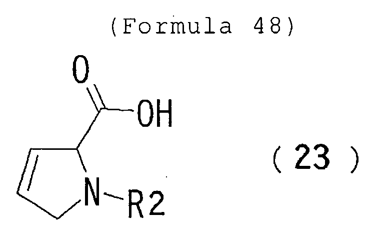

a compound represented by the general formula (24) is generated However, the compound represented by the general formula (23) or the compound represented by the general formula (24) may be chemically changed by performing the treatment (B) so that it is possible for the content of these compounds in the reaction product to be made less than 0.1% by weight.

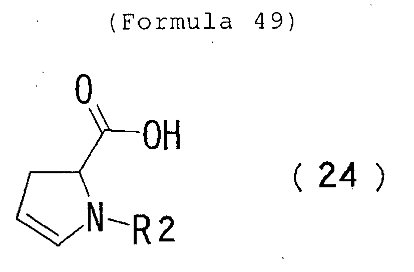

R2 in the general formula (23) and the general formula (24) is defined as R2 in the general formula (9).

As mentioned above, by performing the treatments (A) and (B) on a mixture containing the compounds represented by the general formula (2), the general formula (3) and the general formula (4) and followed by recovering the compound represented by the general formula (5) from the reaction mixture obtained by these treatments, there may be produced the compound represented by the general formula (5) in which the content of the compound represented by the general formula (23) and the compound represented by the general formula (24) are 0.1 % or less by weight, respectively.
Therefore, this method is useful as a method for purifying the compound represented by the general formula (5).
In addition, as mentioned above, by performing the treatments (A) and (B) on a mixture containing the compound represented by the general formula (10), the compound represented by the general formula (3) and the compound represented by the general formula (4) followed by recovering the compound represented by the general formula (12) from the reaction mixture obtained by these treatments, there may be produced the compound represented by the general formula (12) in which the content of the compound represented by the general formula (23) and the compound represented by the general formula (24) is 0.1 % by weight or less, respectively.
Therefore, this method is useful as a method for purifying the compound represented by the general formula (12).

In other words, this method is useful as a method for purifying a compound represented by the general formula (13) in which the treatment (B) is performed on a mixture containing the compound represented by the general formula (13), the compound represented by the general formula (14) and the compound represented by the general formula (15), and subsequently the compound represented by the general formula (13) is recovered. 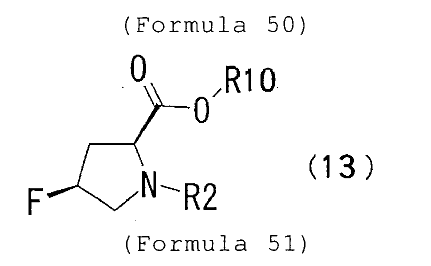 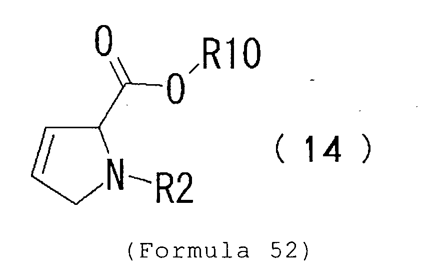 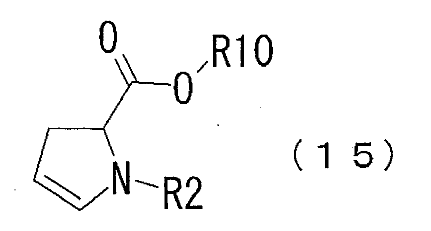 In addition, this method is also useful as a method for purifying a compound represented by the general formula (16) in which the treatments (A) and (B) are performed on a reaction product containing the compound represented by the general formula (13), the compound represented by the general formula (14) and the compound represented by the general formula (15) (with the proviso that the order of performing (A) and (B) may be reversed and (A) is not required where R10 is a hydrogen atom), and subsequently the compound represented by the general formula (16) is recovered.

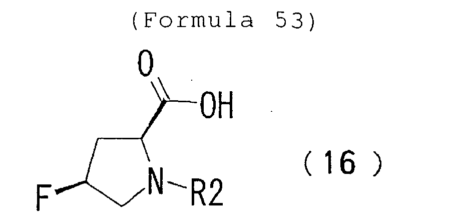

R10 in the general formula (13), the general formula (14) and the general formula (15) represents a hydrogen atom, an unsubstituted or substituted alkyl group having 1 to 6 carbon atoms, an unsubstituted or substituted benzyl group and an unsubstituted or substituted aryl group. The unsubstituted or substituted alkyl group having 1 to 6 carbon atoms, the unsubstituted or substituted benzyl group and the unsubstituted substituted aryl group are defined as R1 in the general formula (1). In addition, R2 in the general formula (13), the general formula (14), the general formula (15) and the general formula (16) is defined as R2 in the general formula (1)

.
This method is useful as a method for purifying a compound represented by the general formula (17) in which the treatment (B) is performed on a mixture containing the compound represented by the general formula (17), the compound represented by the general formula (14) and the compound represented by the general formula (15), and subsequently the compound represented by the general formula (17) is recovered.

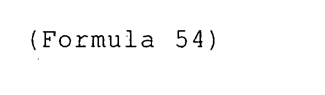

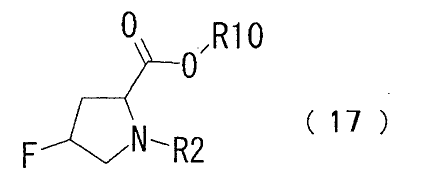

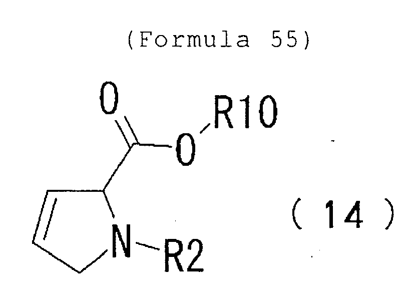

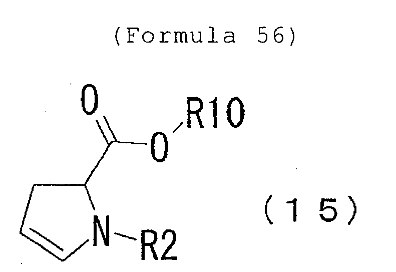

In addition, this method is useful as a method for purifying a compound represented by the general formula (18) in which the treatments (A) and (B) are performed on a reaction product containing the compound represented by the general formula (17), the compound represented by the general formula (14) and the compound represented by the general formula (15) (with the proviso that the order of performing (A) and (B) may be reversed and (A) is not required when R10 is a hydrogen atom), and subsequently the compound represented by the general formula (18) is recovered.

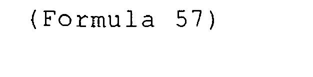

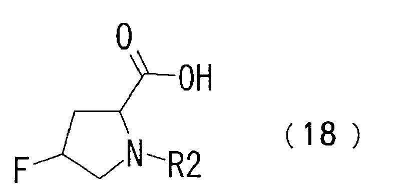

R10 in the general formula (17), the general formula (14) and the general formula (15) represents a hydrogen atom, an unsubstituted or substituted alkyl group having 1 to 6 carbon atoms, an unsubstituted or substituted benzyl group and an unsubstituted or substituted aryl group. The unsubstituted or substituted alkyl group having 1 to 6 carbon atoms, the unsubstituted or substituted benzyl group and the unsubstituted substituted aryl group are defined as R1 in the general formula (9). In addition, R2 in the general formula (17), the general formula (14), the general formula (15) and the general formula (18) is defined as R2 in the general formula (9).
By the above-mentioned method, there may be obtained a compound represented by the general formula (21) in which the content of a compound represented by the general formula (19) and a compound represented by the general formula (20) are less than 0. 1 % by weight, respectively, with respect to the compound represented by the general formula (21). 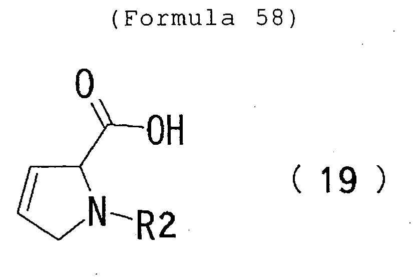 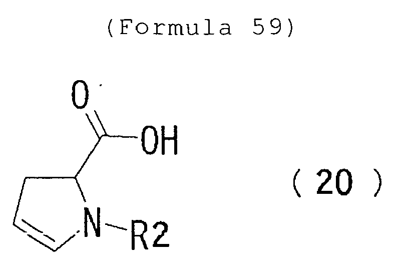 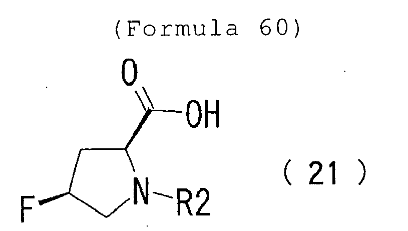 In addition, R2 in the general formula (19), the general formula (20) and the general formula (21) is defined as R2 in the general formula (9).
Further, by the above-mentioned method, there may be obtained a compound represented by the general formula (22) in which the content of a compound represented by the general formula (19) and a compound represented by the general formula (20) are less than 0.1 % by weight, respectively, with respect to the compound represented by the general formula (22). 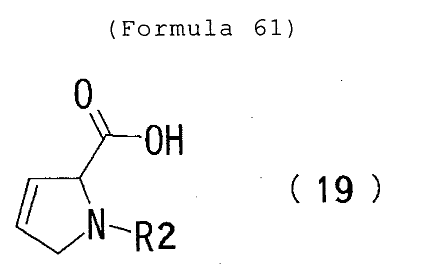 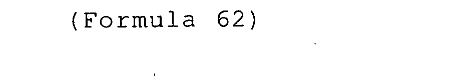 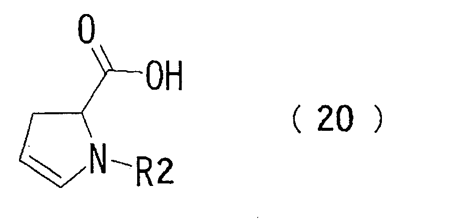 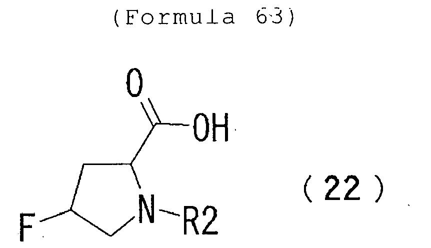 In addition, R2 in the general formula (19), the general formula (20) and the general formula (22) is defined as R2 in the general formula (1).
In this way, by using the production method or purification method of the present invention, there may be obtained a fluorinated proline product useful for materials such as pharmaceuticals, agrochemicals, cosmetics and the like with high efficiency and high yield.
Hereinafter, examples of the present invention will be described, but the technical scope of the present invention will not be limited to these examples.

### (Example 1)

### Production of (4S)-N-tert-butoxycarbonyl-4-fluoro-L-proline (hereinafter, abbreviated as "FBP")

### [1] Production of (4S)-N-tert-butoxycarbonyl-4-fluoro-L-proline methylester (hereinafter, abbreviated as "FBPM") represented by the following formula:

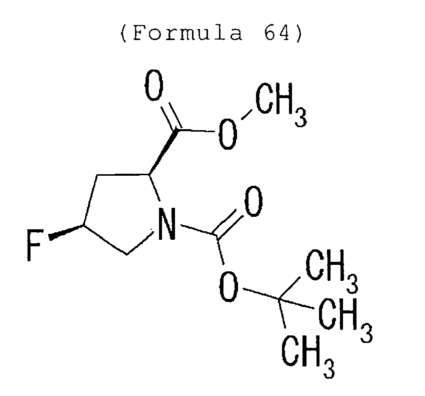

A reaction solution was prepared by suspending (4R)-N-tert-butoxycarbonyl-4-hydroxy-L-proline methylester (hereinafter, abbreviated as "HBPM") (21.4 g) in toluene (107 g) and heated at 75°C with stirring. To the resulting reaction solution was dropwise added 2,2-difluoro-1,3-dimethylimidazolidine (17.7 g) over one hour and the resultant mixture was stirred at the same temperature for 3 hours. After the reaction solution was cooled, a 25 % potassium hydroxide solution (43.3 g) was added to neutralize the solution. The toluene layer was fractionated and was washed with water (53 g) four times. The reaction solution was concentrated under the reduced pressure to obtain a pale yellow syrup (26.1 g) containing FBPM.

Yield amount: 17.5 g (value as converted to FBPM)
Yield rate: 81 % (value against calculated FBPM)
¹N-NMR (CDCl₃, 270 MHz) as FBPM δ: 5.20 (dm, 1H, J = 53 Hz), 4.48 (dd, 1H, J = 9.0, 31 Hz), 3.87 - 3.58 (m, 2H), 3.75 (s, 3H), 2.55 - 2.31 (m, 2H), 1.48 and 1.44 (2s, 9H).
It was confirmed by the HPLC analysis (HPLC analytical condition 1) that the pale yellow syrup (100 % by weight) containing FBPM included, as a by-product, 10 % by weight of N-tert-butoxycarbonyl-3,4-dehydro-L-proline methylester (hereinafter, abbreviated as "3,4-DBPM") represented by the following formula;

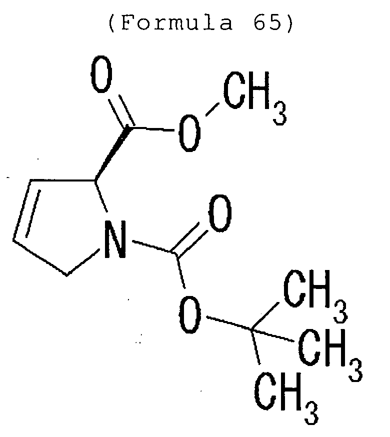

and 1.1 % by weight of
N-tert-butoxycarbonyl-4,5-dehydro-L-proline methylester (hereinafter, abbreviated as "4,5-DBPM") represented by the following formula.

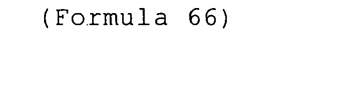

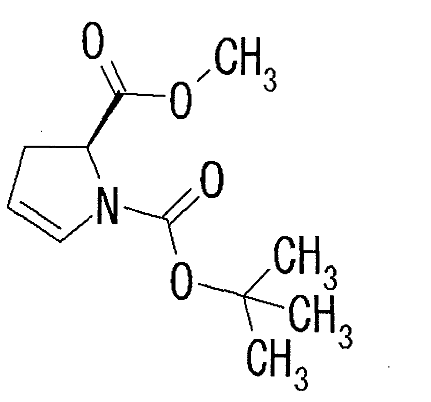

### [HPLC Analytical Condition 1]

| | |
|---|---|
| Column: | YMC-PACK ODS AM-312 |
| Eluent: | Acetonitrile/water (50/50) |
| Flow Rate: | 1 ml/min |
| Detection Wavelength: | UV 210 nm |

[2] Production of FBP represented by the following formula

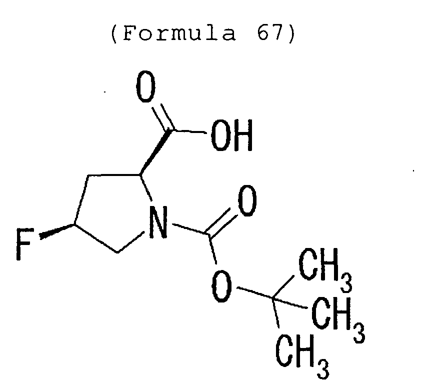

Subsequently, a reaction solution was prepared by adding an aqueous solution (67 g) of 8 % sodium hydroxide to the pale yellow syrup (22.7 g) (containing 15.2 g of FBPM) obtained in the above [1] and stirred at 40°C for 2 hours. The resulting reaction solution was cooled to 5°C, and its pH was adjusted to 5.0 with 18 % hydrochloric acid.
To the reaction solution was dropwise added an aqueous solution (12.3 g) of 11.7 % sodium hypochlorite at a reaction temperature of 5°C and the resulting solution was stirred for 30 min. It was confirmed by the HPLC analysis (HPLC analytical condition 2) that both N-tert-butoxycarbonyl-3,4-dehydro-L-proline (abbreviated as "3,4-DBP") and N-tert-butoxycarbonyl-4,5-dehydro-L-proline (hereinafter, abbreviated as "4,5-DBP") which are impurities were less than 0.1 % by weight with respect to FBP (100 % by weight). The pH of the reaction solution was adjusted to 9.0 with an aqueous solution of 10 % sodium hydroxide and washed with toluene (75 g) twice. The pH of the resulting aqueous layer was adjusted to 2.0 with 18 % hydrochloric acid and extraction was performed with ethyl acetate (150 g, twice). The ethyl acetate layer was dried with anhydrous magnesium sulfate and concentrated under the reduced pressure. Toluene (75 g) was added to the resulting residue to obtain FBP as white crystals.

Yield amount: 11.8 g (value as converted to FBP)
Yield rate: 82 % (value against calculated FBP)
Melting Point: 161 to 162°C
¹N-NMR (CDCl₃, 270 MHz) δ: 9.60 (bs, 1H), 5.21 (dm, 1H, J = 52 Hz), 4.57 - 4.44 (m, 1H), 3. 93 - 3.52 (m, 2H), 2.79 - 2.24 (m, 2H), 1.49 and 1.44 (2s, 9H).
The HPLC analysis (HPLC analytical condition 2) showed that the content of 3,4-DBP represented by the following formula was 0.01% by weight or less in the white crystals,

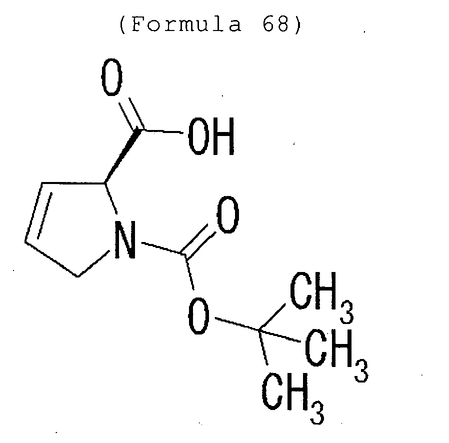

and also the content of 4,5-DBP represented by the following formula was 0.01 % by weight or less.

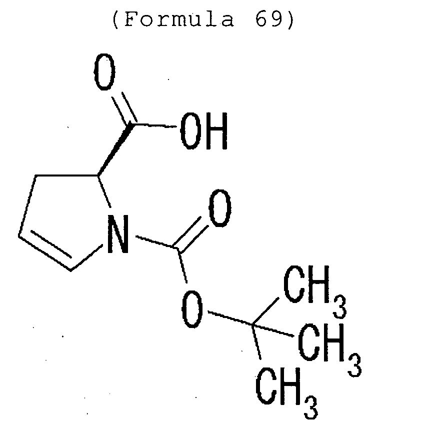

### [HPLC Analytical Condition 2]

Column: YMC-PACK ODS AM-312
Eluent: Acetonitrile/10 mM NaH₂PO₄-H₃PO₄ (pH 2.5) (20/80)
Flow Rate: 1 ml/min
Detection Wavelength: UV 210 nm

### (Example 2)

### Production of FBP

The reaction in [1] of Example 1 was carried out to obtain a pale yellow syrup (26.1 g) containing 17.5 g (yield rate: 81 %) of FBPM (value against calculated FBPM). The pale yellow syrup containing FBPM included 10 % by weight of 3, 4-DBPM with respect to FBPM and 1.1 % by weight of 4,5-DBPM with respect to FBPM.

FBP as white crystals was obtained by treating the pale yellow syrup (22.7 g) containing 15.2 g as FBPM) in the same manner as the reaction in [2] in Example 1 except for switching the aqueous solution of 11.7 % sodium hypochlorite to bromine (3.73 g).
Yield amount: 11.8 g (value as converted to FBP)
Yield rate: 82 % (value against calculated FBP)
The melting point and ¹N-NMR (CDCl₃, 270 MHz) were the same as those in Example 1.

It was confirmed by the HPLC analysis that both 3,4-DBP and 4, 5-DBP which are impurities were 0.01 % by weight or less. Meanwhile, the HPLC analytical condition was the same as that in Example 1.

### (Example 3)

### Production of FBP

The reaction in [1] of Example 1 was carried out to obtain a pale yellow syrup (26.1 g) containing 17.5 g (yield rate: 81 %) of FBPM (value against calculated FBPM). The pale yellow syrup containing FBPM included 10 % by weight of 3, 4-DBPM with respect to FBPM and 1.1 % by weight of 4,5-DBPM with respect to FBPM.

A reaction solution was prepared by dissolving the pale yellow syrup (1.48 g) (containing 10 g of FBPM) in 10 g of dichloromethane and cooled to 5°C, to which was added dropwise bromine (246 mg). The reaction solution was raised to room temperature and stirred for 4 hours. It was confirmed by the HPLC analysis (HPLC analytical condition 1) that 3,4-DBPM and 4,5-DBPM which are impurities were less than 0.1 % by weight with respect to FBPM. The reaction solution was concentrated under the reduced pressure and the resulting residue was dissolved in methanol (2 g), followed by adding an aqueous solution (8.8 g) of 8 % sodium hydroxide and stirring at 40°C for 2 hours. The reaction solution was cooled to room temperature and washed with toluene (10 g) twice. The pH of the resulting aqueous layer was adjusted to 2.0 with 18 % hydrochloric acid and the resulting solution was extracted with ethyl acetate (15 g). The ethyl acetate layer was dried with anhydrous magnesium sulfate and concentrated under the reduced pressure. Toluene (5 g) was added to the resulting residue to crystallize and obtain FBP as white crystals.

| | |
|---|---|
| Yield amount: | 754 mg (value as converted to FBP) |
| Yield rate: | 80 % (value against calculated FBP) |

The melting point and ¹N-NMR (CDCl₃, 270 MHz) were the same as those in Example 1.
It was confirmed by the HPLC analysis that 3,4-DBP was 0.02 % by weight and 4,5-DBP was 0.01 % by weight or less in white crystals. Meanwhile, the HPLC analytical condition was the same as that in Example 1.

### (Example 4)

### Production of FBP

The reaction in [1] of Example 1 was carried out to obtain a pale yellow syrup (26.1 g) containing 17.5 g (yield rate: 81 %) of FBPM (value against calculated FBPM). The pale yellow syrup containing FBPM included 10 % by weight of 3, 4-DBPM with respect to FBPM and 1.1 % by weight of 4, 5-DBPM with respect to FBPM.
FBP as white crystals was obtained by treating the pale yellow syrup (2.27 g) (containing 1.52 g as FBPM) in the same manner as the reaction 2 in Example 1 except for switching the aqueous solution of 11.7 % sodium hypochlorite to N-bromosuccinimide (0.53 g).

| | |
|---|---|
| Yield amount: | 0.92 g (value as converted to FBP) |
| Yield rate: | 85 % (value against calculated FBP) |

The melting point and ¹N-NMR (CDCl₃, 270 MHz) were the same as those in Example 1.

It was confirmed by the HPLC analysis that both 3,4-DBP and 4,5-DBP in white crystals were 0.01 % by weight or less. Meanwhile, the HPLC analytical condition was the same as that in Example 1.

### (Example 5)

### Production of FBP

A reaction solution was prepared by dissolving HBPM (5.75 g) in dichloromethane (50 g), and thereto was dropwise added (diethylamino) sulfur trifluoride (DAST) (5.70 g) at 5°C. The reaction solution was stirred at 5°C for 12 hours, followed by adding dropwise thereto a saturated aqueous solution (100 g) of sodium hydrogencarbonate. Dichloromethane (100 g) was added to the resulting reaction solution while stirring and an organic layer was fractionated. The organic layer was washed with water (100 g) and dried with anhydrous magnesium sulfate. The resulting organic layer was vacuum concentrated to obtain a pale yellow syrup (5.68 g) containing FBPM.

| | |
|---|---|
| Yield amount: | 4.26 g (value as converted to FBPM) |
| Yield rate: | 73 % (value against calculated FBPM) |

The ¹N-NMR (CDCl₃, 270 MHz) as FBPM was the same as that in Example 1.

The pale yellow syrup containing FBPM included 4.0 % by weight of 3,4-DBPM with respect to FBPM and 0.6 % by weight of 4,5-DBPM with respect to FBPM, as a by-product, respectively. Meanwhile, the HPLC analytical condition was the same as that in Example 1.

Subsequently, a reaction solution was prepared by adding an aqueous solution (6.7 g) of 8 % sodium hydroxide to the pale yellow syrup (2.03 g) (containing 1.52 g of FBPM) obtained in the above and stirred at 40°C for 2 hours. The resulting reaction solution was cooled to 5°C, and adjusted the pH to 5.0 with 18 % hydrochloric acid. To the reaction solution was dropwise added an aqueous solution (0.50 g) of 11.7 % sodium hypochlorite at a reaction temperature of 5°C and the resultant reaction solution was stirred for 30 min. The pH of the reaction solution was adjusted to 9.0 with an aqueous solution of 10 % sodium hydroxide and washed with toluene (10 g) twice. The pH of the resulting aqueous layer was adjusted to 2.0 with 18 % hydrochloric acid and the resultant solution was.extracted with ethyl acetate (15 g, twice). The ethyl acetate layer was dried with anhydrous magnesium sulfate and concentrated under the reduced pressure. Toluene (7.5 g) was added to the resulting residue to obtain FBP as white crystals.

| | |
|---|---|
| Yield amount: | 1.18 g (value as converted to FBP) |
| Yield rate: | 82 % (value against calculated FBP) |

The melting point and ¹N-NMR (CDCl₃, 270 MHz) were the same as those in Example 1.

It was confirmed by the HPLC analysis that both 3,4-DBP and 4,5-DBP in white crystals were 0.01 % by weight or less. Meanwhile, the HPLC analytical condition was the same as that in Example 1.

### [Comparative example] Production of FBP

A reaction solution was prepared by dissolving HBPM (5.75 g) in dichloromethane (50 g), and thereto was added dropwise (diethylamino)sulfur trifluoride (DAST) (5.70 g) at 5°C. After the reaction solution was stirred at 5°C for 12 hours, a saturated aqueous solution (100 g) of sodium hydrogencarbonate was added dropwise to the reaction solution. Dichloromethane (100 g) was added to the resulting reaction solution while stirring and an organic layer was fractionated. The organic layer was washed with water (100 g) and dried with anhydrous magnesium sulfate. The resulting organic layer was concentrated under the reduced pressure to obtain a pale yellow syrup (5.68) containing FBPM.

| | |
|---|---|
| Yield amount: | 4.26 g (value as converted to FBPM) |
| Yield rate: | 73 % (value against calculated FBPM) |

The ¹N-NMR (CDCl₃, 270 MHz) as FBPM was the same as that in Example 1.

The pale yellow syrup containing FBPM included 4.0 % by weight of 3,4-DBPM with respect to FBPM and 0.6 % by weight of 4,5-DBPM with respect to FBPM. Meanwhile, the HPLC analytical condition was the same as that in Example 1.

Subsequently, a reaction solution was prepared by adding an aqueous solution (6.7 g) of 8 % sodium hydroxide to the pale yellow syrup (1.52g as the content of FBPM) containing FBPM obtained in the above and stirred at 40°C for 2 hours. The resulting reaction solution was cooled to room temperature, adjusted the pH to 2.0 with 18 % hydrochloric acid, and extracted with ethyl acetate (15g, twice) . The ethyl acetate layer was dried with anhydrous magnesium sulfate and concentrated under the reduced pressure. Toluene (7.5 g) was added to the resulting residue to obtain FBP as white crystals.

| | |
|---|---|
| Yield amount: | 1.09 g (value as converted to FBP) |
| Yield rate: | 75 % (value against calculated FBP) |
| Melting point: | 158 to 160°C |

The peak value of ¹N-NMR (CDCl₃, 270 MHz) derived from FBP was the same as that in Example 1.

It was confirmed by the HPLC analysis that the white crystals contained 1.0 % by weight of 3, 4-DBP and 0.2 % by weight of 4,5-DBP which are impurities. Meanwhile, the HPLC analysis condition was the same as that in Example 1.

## Claims

1. A method for producing a compound represented by the general formula (2), comprising:
reacting a compound represented by the general formula (1) with a fluorination agent to obtain a reaction product containing a compound represented by the general formula (2), a compound represented by the general formula (3) and a compound represented by the general formula (4); and
bringing chlorine, bromine, iodine, hypochlorous acid, hypochlorite, hypobromous acid, hypobromite or N-halogenosuccinimide into action on the reaction product to chemically change the compounds represented by the general formula (3) and the general formula (4), wherein the general formulas (1), (2), (3) and (4) represent as the following, respectively: 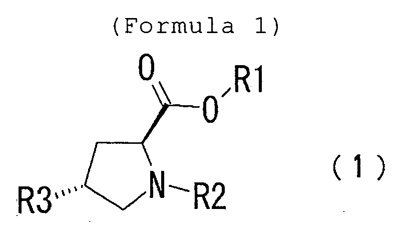 (in the formula, R1 represents an unsubstituted or substituted alkyl group having 1 to 6 carbon atoms, an unsubstituted or substituted benzyl group or an unsubstituted or substituted aryl group, R2 represents an unsubstituted or substituted alkyl group having 1 to 6 carbon atoms, an unsubstituted or substituted benzyl group, an unsubstituted or substituted aryl group or -CO-X, and X represents an unsubstituted or substituted alkyl group having 1 to 6 carbon atoms, a trifluoromethyl group, an unsubstituted or substituted aryl group, an unsubstituted or substituted alkoxy group having 1 to 6 carbon atoms, an unsubstituted or substituted benzyloxy group, or an unsubstituted or substituted fluorenylmethoxy group, and R3 represents a hydroxyl group, an unsubstituted or substituted alkylsulfonyloxy group having 1 to 7 carbon atoms, an unsubstituted or substituted arylsulfonyloxy group or a trifluoromethanesulfonyloxy group), 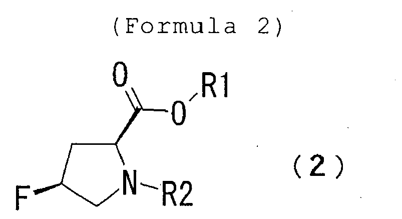 (in the formula, R1 and R2 are defined as in the general formula (1)), 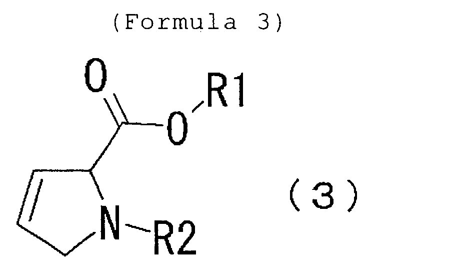 (in the formula, R1 and R2 are defined as in the general formula (1)), and 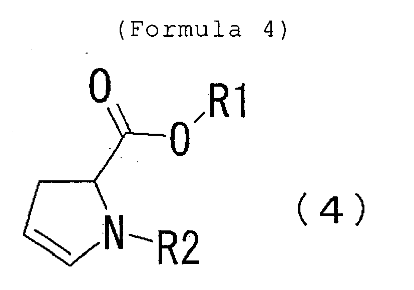 (in the formula, R1 and R2 are defined as in the general formula (1)).

2. A method for producing a compound represented by the general formula (5), comprising:
reacting a compound represented by the general formula (1) with a fluorination agent to obtain a reaction product containing a compound represented by the general formula (2), a compound represented by the general formula (3) and a compound represented by the general formula (4); and
performing the following treatments (A) and (B) on the reaction product (the order of performing (A) and (B) may be reversed); and
recovering the compound represented by the general formula (5):
(A) a treatment of converting the carboxylic acid ester portion containing R1 in the general formula (2), the general formula (3) and the general formula (4) to a carboxylic acid or a salt thereof, and
(B) a treatment of bringing chlorine, bromine, iodine, hypochlorous acid, hypochlorite, hypobromous acid, hypobromite or N-halogenosuccinimide into action on the reaction product to chemically change the compounds represented by the general formula (3) and the general formula (4), wherein the general formulas (1), (2), (3), (4) and (5) represent as the following, respectively: 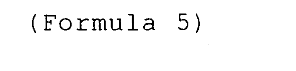 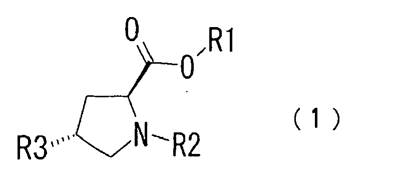 (in the formula, R1 represents an unsubstituted or substituted alkyl group having 1 to 6 carbon atoms, an unsubstituted or substituted benzyl group or an unsubstituted or substituted aryl group, R2 represents an unsubstituted or substituted alkyl group having 1 to 6 carbon atoms, an unsubstituted or substituted benzyl group, an unsubstituted or substituted aryl group or -CO-X, and X represents an unsubstituted or substituted alkyl group having 1 to 6 carbon atoms, a trifluoromethyl group, an unsubstituted or substituted aryl group, an unsubstituted or substituted alkoxy group having 1 to 6 carbon atoms, an unsubstituted or substituted benzyloxy group, or an unsubstituted or substituted fluorenylmethoxy group, and R3 represents a hydroxyl group, an unsubstituted or substituted alkylsulfonyloxy group having 1 to 7 carbon atoms, an unsubstituted or substituted arylsulfonyloxy group or a trifluoromethanesulfonyloxy group), 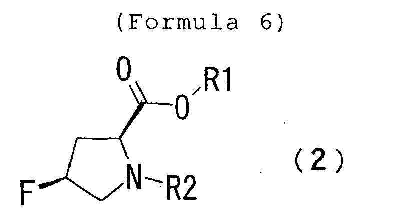 (in the formula, R1 and R2 are defined as in the general formula (1)), 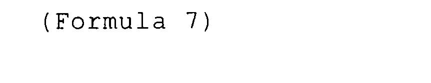 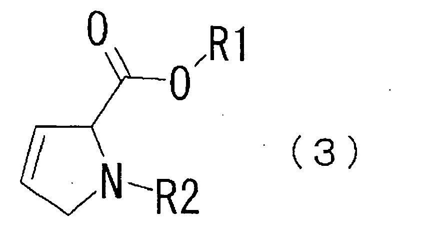 (in the formula, R1 and R2 are defined as in the general formula (1)). 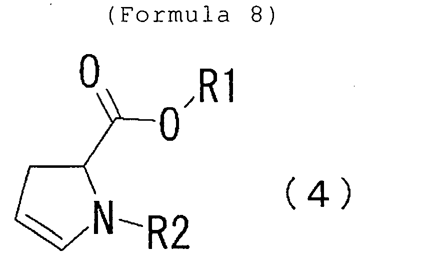 (in the formula, R1 and R2 are defined as in the general formula (1)), and 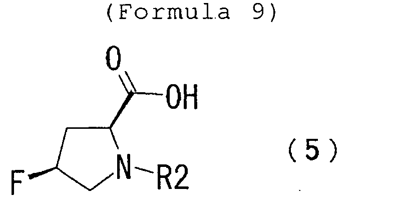 (in the formula, R2 is defined as in the general formula (1)).

3. The method according to claim 1 or 2, wherein R3 of said general formula (1) is a hydroxyl (OH) group.

4. The method according to claim 1 or 2, wherein 0.1 to 10 molar equivalents of chlorine, bromine, iodine, hypochlorous acid, hypochlorite, hypobromous acid, hypobromite or N-halogenosuccinimide is brought into reaction, based on 1 molar equivalent in total of the compound represented by the general formula (3) and the compound represented by the general formula (4).

5. The method according to claim 1 or 2, wherein the fluorination agent is a compound represented by the general formula (6) or a compound represented by the general formula (7), wherein the general formulas (6) and (7) represent as the following, respectively: 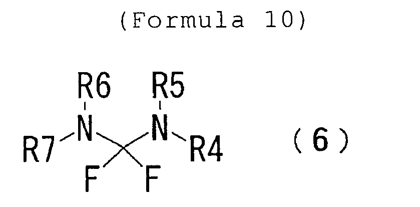 (in the formula, R4, R5, R6 and R7 represent an unsubstituted or substituted alkyl group having 1 to 6 carbon atoms, an unsubstituted or substituted benzyl group or an unsubstituted or substituted aryl group and they may be the same or different, R5 and R6 may be linked together to form a five-membered ring or a six-membered ring, and R4 and R5, or R6 and R7 may be linked together to form a ring), and 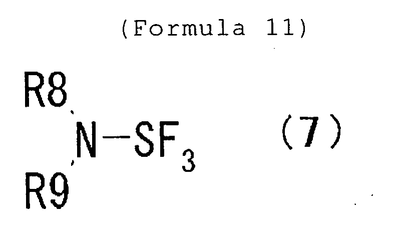 (in the formula, R8 and R9 represent an unsubstituted or substituted alkyl group having 1 to 6 carbon atoms, an unsubstituted or substituted benzyl group or an unsubstituted or substituted aryl group and they may be the same or different, R8 and R9 may be linked together to form a ring).

6. The method according to claim 1 or 2, wherein the fluorination agent is 2,2-difluoro-1,3-dimethylimidazolidine represented by the formula (8) or (diethylamino)sulfur trifluoride, wherein the formula (8) represents:

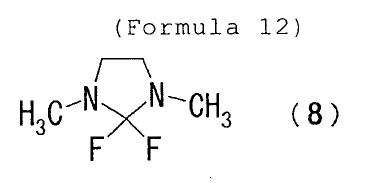

7. A method for producing a compound represented by the general formula (10), comprising:
reacting a compound represented by the general formula (9) with a fluorination agent to obtain a reaction product containing a compound represented by the general formula (10), a compound represented by the general formula (3) and a compound represented by the general formula (4); and
bringing chlorine, bromine, iodine, hypochlorous acid, hypochlorite, hypobromous acid, hypobromite or N-halogenosuccinimide into action on the reaction product to chemically change the compounds represented by the general formula (3) and the general formula (4), wherein the general formulas (9), (10), (3) and (4) represent as the following, respectively: 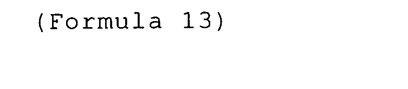 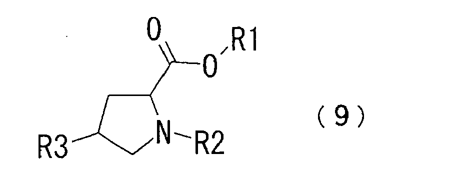 (in the formula, R1 represents an unsubstituted or substituted alkyl group having 1 to 6 carbon atoms, an unsubstituted or substituted benzyl group or an unsubstituted or substituted aryl group, R2 represents an unsubstituted or substituted alkyl group having 1 to 6 carbon atoms, an unsubstituted or substituted benzyl group, an unsubstituted or substituted aryl group or -CO-X, and X represents an unsubstituted or substituted alkyl group having 1 to 6 carbon atoms, a trifluoromethyl group, an unsubstituted or substituted aryl group, an unsubstituted or substituted alkoxy group having 1 to 6 carbon atoms, an unsubstituted or substituted benzyloxy group, or an unsubstituted or substituted fluorenylmethoxy group, and R3 represents a hydroxyl group, an unsubstituted or substituted alkylsulfonyloxy group having 1 to 7 carbon atoms, an unsubstituted or substituted arylsulfonyloxy group or a trifluoromethanesulfonyloxy group), 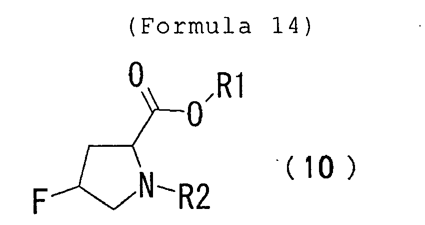 (in the formula, R1 and R2 are defined as in the general formula (9)), 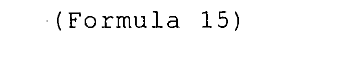 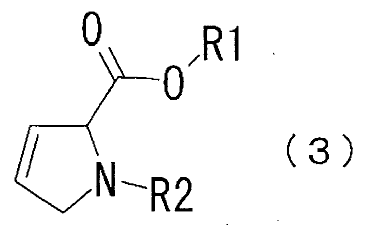 (in the formula, R1 and R2 are defined as in the general formula (9)), and 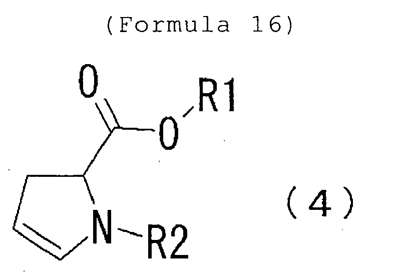 (in the formula, R1 and R2 are defined as in the general formula (9)).

8. A method for producing a compound represented by the general formula (12), comprising:
reacting a compound represented by the formula (9) with a fluorination agent obtain a reaction product containing a compound represented by the general formula (10), a compound represented by the general formula (3) and a compound represented by the general formula (4); and performing the following treatments (A) and (B) on the reaction product (the order of performing (A) and (B) may be reversed); and
recovering the compound represented by the general formula (12):
(A) a treatment of converting the carboxylic acid ester portion containing R1 in the general formula (10), the general formula (3) and the general formula (4) to a carboxylic acid or a salt thereof, and
(B) a treatment of bringing chlorine, bromine, iodine, hypochlorous acid, hypochlorite, hypobromous acid, hypobromite or N-halogenosuccinimide into action on the reaction product to chemically change the compounds represented by the general formula (3) and the general formula (4), wherein the general formulas (9), (10), (3), (4) and (12) represent as the following, respectively: 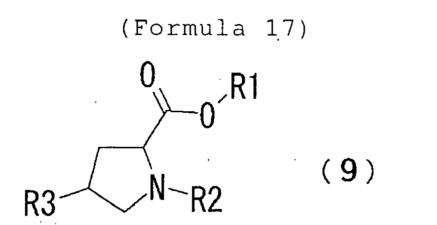 (in the formula, R1 represents an unsubstituted or substituted alkyl group having 1 to 6 carbon atoms, an unsubstituted or substituted benzyl group or an unsubstituted or substituted aryl group, R2 represents an unsubstituted or substituted alkyl group having 1 to 6 carbon atoms, an unsubstituted or substituted benzyl group, an unsubstituted or substituted aryl group or -CO-X, and X represents an unsubstituted or substituted alkyl group having 1 to 6 carbon atoms, a trifluoromethyl group, an unsubstituted or substituted aryl group, an unsubstituted or substituted alkoxy group having 1 to 6 carbon atoms, an unsubstituted or substituted benzyloxy group, or an unsubstituted or substituted fluorenylmethoxy group, and R3 represents a hydroxyl group, an unsubstituted or substituted alkylsulfonyloxy group having 1 to 7 carbon atoms, an unsubstituted or substituted arylsulfonyloxy group or a trifluoromethanesulfonyloxy group), 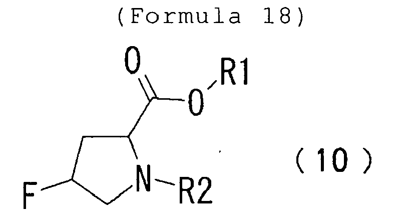 (in the formula, R1 and R2 are defined as in the general formula (9)), 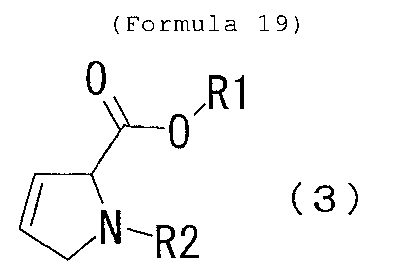 (in the formula, R1 and R2 are defined as in the general formula (9)), 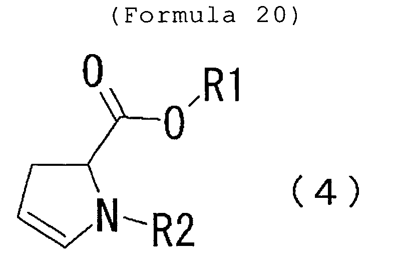 (in the formula, R1 and R2 are defined as in the general formula (9)), and 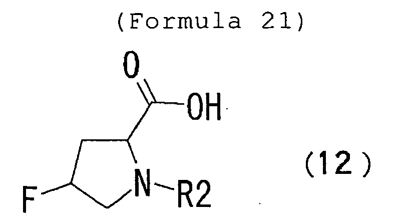 (in the formula, R2 is defined as in the general formula (9)).

9. The method according to claim 7 or 8, wherein R3 of said general formula (9) is a hydroxyl (OH) group.

10. The method according to claim 7 or 8, wherein 0.1 to 10 molar equivalents of chlorine, bromine, iodine, hypochlorous acid, hypochlorite, hypobromous acid, hypobromite or N-halogenosuccinimide is brought into reaction, based on 1 molar equivalent in total of the compound represented by the general formula (3) and the compound represented by the general formula (4).

11. The method according to claim 7 or 8, wherein the fluorination agent is a compound represented by the general formula (6) or a compound represented by the general formula (7), wherein the general formulas (6) and (7) represent as the following, respectively: 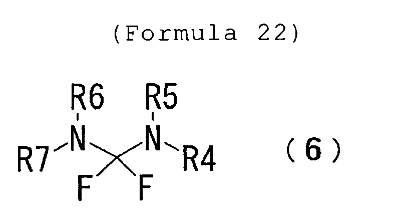 (in the formula, R4, R5, R6 and R7 represent an unsubstituted or substituted alkyl group having 1 to 6 carbon atoms, an unsubstituted or substituted benzyl group or an unsubstituted or substituted aryl group and they may be the same or different, R5 and R6 may be linked together to form a five-membered ring or a six-membered ring, and R4 and R5, or R6 and R7 may be linked together to form a ring), and 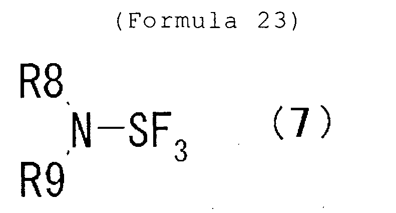 (in the general formula, R8 and R9 represent an unsubstituted or substituted alkyl group having 1 to 6 carbon atoms, an unsubstituted or substituted benzyl group or an unsubstituted or substituted aryl group and they may be the same or different, and R8 and R9 may be linked together to form a ring).

12. The method according to claim 7 or 8, wherein the fluorination agent is 2,2-difluoro-1,3-dimethylimidazolidine represented by the formula (8) or (diethylamino)sulfur trifluoride, wherein the formula (8) represents:

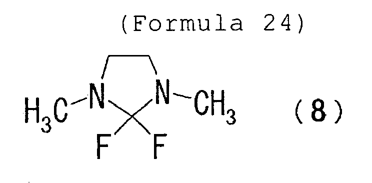

13. A method for purifying a compound represented by the general formula (13), comprising:
performing the following treatments (A) and (B) on a mixture containing a compound represented by the general formula (13), a compound represented by the general formula (14) and a compound represented by the general formula (15) (with the proviso that the order of performing (A) and (B) may be reversed, (A) is not required where R10 is a hydrogen atom and (A) is performed as needed where R10 is other than a hydrogen atom) ; and
recovering the compound represented by the general formula (13):
(A) a treatment of converting the carboxylic acid ester portion containing R10 in the general formula (13), the general formula (14) and the general formula (15) to a carboxylic acid or a salt thereof, and
(B) a treatment of bringing chlorine, bromine, iodine,
hypochlorous acid, hypochlorite, hypobromous acid, hypobromite or N-halogenosuccinimide into action on the mixture to chemically change the compounds represented by the general formula (14) and the general formula (15), wherein the general formulas (13), (14) and (15) represent as the following, respectively: 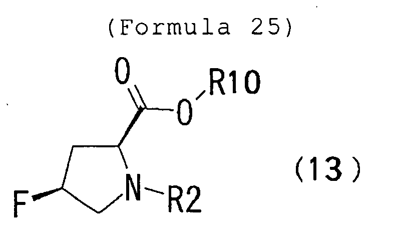 (in the formula, R10 represents a hydrogen atom, an unsubstituted or substituted alkyl group having 1 to 6 carbon atoms, an unsubstituted or substituted benzyl group or an unsubstituted or substituted aryl group, R2 represents an unsubstituted or substituted alkyl group having 1 to 6 carbon atoms, an unsubstituted or substituted benzyl group, an unsubstituted or substituted aryl group or -CO-X, and X represents an unsubstituted or substituted alkyl group having 1 to 6 carbon atoms, a trifluoromethyl group, an unsubstituted or substituted aryl group, an unsubstituted or substituted alkoxy group having 1 to 6 carbon atoms, an unsubstituted or substituted benzyloxy group, or an unsubstituted or substituted fluorenylmethoxy group), 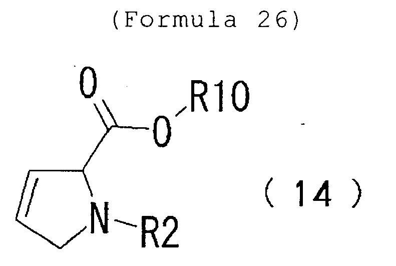 (in the formula, R2 and R10 are defined as in the general formula (13)), and 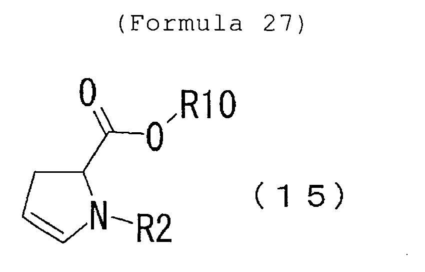 (in the formula, R2 and R10 are defined as in the general formula (13)).

14. The method according to claim 13, wherein in said treatment (B), 0.1 to 10 molar equivalents of chlorine, bromine, iodine, hypochlorous acid, hypochlorite, hypobromous acid, hypobromite or N-halogenosuccinimide is brought into reaction, based on 1 molar equivalent in total of the compound represented by the general formula (14) and the compound represented by the general formula (15).

15. A method for purifying a compound represented by the general formula (17), comprising:
performing the following treatments (A) and (B) on a mixture containing a compound represented by the general formula (17), a compound represented by the general formula (14) and a compound represented by the general formula (15) (with the proviso that the order of performing (A) and (B) may be reversed, (A) is not required where R10 is a hydrogen atom and (A) is performed as needed where R10 is other than a hydrogen atom) ; and
recovering the compound represented by the general formula (17):
(A) a treatment of converting the carboxylic acid ester portion containing R10 in the general formula (17), the general formula (14) and the general formula (15) to a carboxylic acid or a salt thereof, and
(B) a treatment of bringing chlorine, bromine, iodine, hypochlorous acid, hypochlorite, hypobromous acid, hypobromite or N-halogenosuccinimide into action on the mixture to chemically change the compounds represented by the general formula (14) and the general formula (15), wherein the general formulas (17), (14) and (15) represent as the following, respectively: 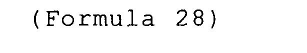 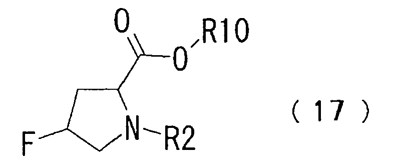 (in the formula, R10 represents a hydrogen atom, an unsubstituted or substituted alkyl group having 1 to 6 carbon atoms, an unsubstituted or substituted benzyl group or an unsubstituted or substituted aryl group, R2 represents an unsubstituted or substituted alkyl group having 1 to 6 carbon atoms, an unsubstituted or substituted benzyl group, an unsubstituted or substituted aryl group or -CO-X, and X represents an unsubstituted or substituted alkyl group having 1 to 6 carbon atoms, a trifluoromethyl group, an unsubstituted or substituted aryl group, an unsubstituted or substituted alkoxy group having 1 to 6 carbon atoms, an unsubstituted or substituted benzyloxy group, or an unsubstituted or substituted fluorenylmethoxy group), 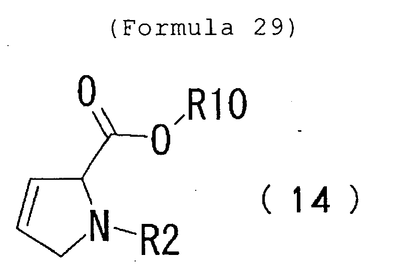 (In the formula, R2 and R10 are defined as in said general formula (17)), and 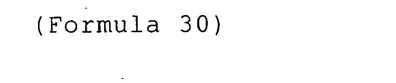 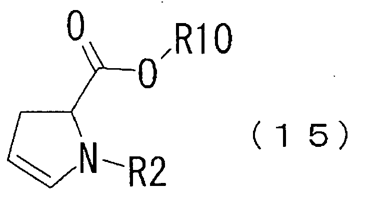 (in the formula, R2 and R10 are defined as in said general formula (17)).

16. The method according to claim 15, wherein in said treatment (B), 0.1 to 10 molar equivalents of chlorine, bromine, iodine, hypochlorous acid, hypochlorite, hypobromous acid, hypobromite or N-halogenosuccinimide is brought into reaction, based on 1 molar equivalent in total of the compound represented by the general formula (14) and the compound represented by the general formula (15).

17. A compound represented by the general formula (21), wherein the content of a compound represented by the general formula (19) and the content of a compound represented by the general formula (20) each are less than 0.1 % by weight with respect to the compound represented by the general formula (21), wherein the general formulas (19), (20) and (21) represent as the following, respectively: 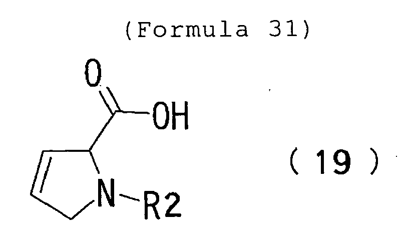 (in the formula, R2 represents an unsubstituted or substituted alkyl group having 1 to 6 carbon atoms, an unsubstituted or substituted benzyl group, an unsubstituted or substituted aryl group or -CO-X, and X represents an unsubstituted or substituted alkyl group having 1 to 6 carbon atoms, a trifluoromethyl group, an unsubstituted or substituted aryl group, an unsubstituted or substituted alkoxy group having 1 to 6 carbon atoms, an unsubstituted or substituted benzyloxy group or an unsubstituted or substituted fluorenylmethoxy group), 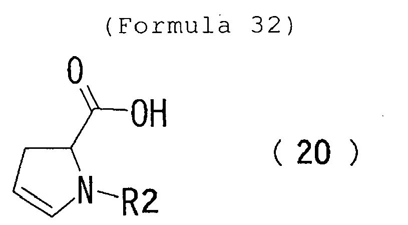 (in the formula, R2 represents an unsubstituted or substituted alkyl group having 1 to 6 carbon atoms, an unsubstituted or substituted benzyl group, an unsubstituted or substituted aryl group or -CO-X, and X represents an unsubstituted or substituted alkyl group having 1 to 6 carbon atoms, a trifluoromethyl group, an unsubstituted or substituted aryl group, an unsubstituted or substituted alkoxy group having 1 to 6 carbon atoms, an unsubstituted or substituted benzyloxy group or an unsubstituted or substituted fluorenylmethoxy group), and 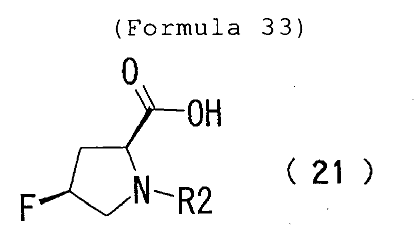 (in the formula, R2 represents an unsubstituted or substituted alkyl group having 1 to 6 carbon atoms, an unsubstituted or substituted benzyl group, an unsubstituted or substituted aryl group or -CO-X, and X represents an unsubstituted or substituted alkyl group having 1 to 6 carbon atoms, a trifluoromethyl group, an unsubstituted or substituted aryl group, an unsubstituted or substituted alkoxy group having 1 to 6 carbon atoms, an unsubstituted or substituted benzyloxy group or an unsubstituted or substituted fluorenylmethoxy group).

18. A compound represented by the general formula (22), wherein the content of a compound represented by the general formula (19) and the content of a compound represented by the general formula (20) each are less than 0.1 % by weight with respect to the compound represented by the general formula (22), wherein the general formulas (19), (20) and (22) represent as the following, respectively: 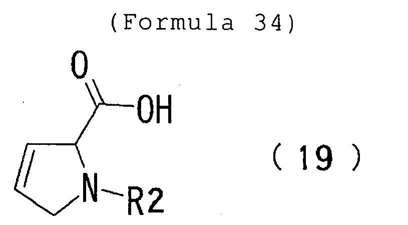 (in the formula, R2 represents an unsubstituted or substituted alkyl group having 1 to 6 carbon atoms, an unsubstituted or substituted benzyl group, an unsubstituted or substituted aryl group or -CO-X, and X represents an unsubstituted or substituted alkyl group having 1 to 6 carbon atoms, a trifluoromethyl group, an unsubstituted or substituted aryl group, an unsubstituted or substituted alkoxy group having 1 to 6 carbon atoms, an unsubstituted or substituted benzyloxy group or an unsubstituted or substituted fluorenylmethoxy group), 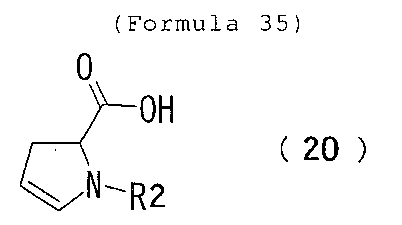 (in the formula, R2 represents an unsubstituted or substituted alkyl group having 1 to 6 carbon atoms, an unsubstituted or substituted benzyl group, an unsubstituted or substituted aryl group or -CO-X, and X represents an unsubstituted or substituted alkyl group having 1 to 6 carbon atoms, a trifluoromethyl group, an unsubstituted or substituted aryl group, an unsubstituted or substituted alkoxy group having 1 to 6 carbon atoms, an unsubstituted or substituted benzyloxy group or an unsubstituted or substituted fluorenylmethoxy group), and 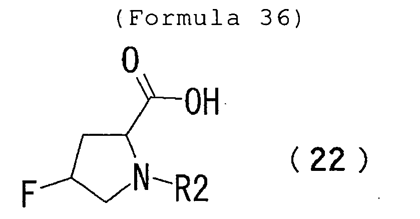 (in the formula, R2 represents an unsubstituted or substituted alkyl group having 1 to 6 carbon atoms, an unsubstituted or substituted benzyl group, an unsubstituted or substituted aryl group or -CO-X, and X represents an unsubstituted or substituted alkyl group having 1 to 6 carbon atoms, a trifluoromethyl group, an unsubstituted or substituted aryl group, an unsubstituted or substituted alkoxy group having 1 to 6 carbon atoms, an unsubstituted or substituted benzyloxy group or an unsubstituted or substituted fluorenylmethoxy group).
